(19) Europäisches Patentamt
European Patent Office
Office européen des brevets

(11) EP 4 730 087 A1

(12) EUROPEAN PATENT APPLICATION
published in accordance with Art. 153(4) EPC

(43) Date of publication:
22.04.2026 Bulletin 2026/17

(51) International Patent Classification (IPC):
G06F 3/01 (2006.01)       H01Q 1/27 (2006.01)
A61B 5/00 (2006.01)       A61B 5/024 (2006.01)
G06F 1/16 (2006.01)       H01Q 5/335 (2015.01)
H01Q 5/10 (2015.01)

(21) Application number: 25834842.4

(22) Date of filing: 22.08.2025

(86) International application number:
PCT/KR2025/012793

(87) International publication number:
WO 2026/049429 (05.03.2026 Gazette 2026/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 27.08.2024 KR 20240115449
16.10.2024 KR 20240141674

(71) Applicant: Samsung Electronics Co., Ltd.
Suwon-si, Gyeonggi-do 16677 (KR)

(72) Inventors:
• CHO, Namjun
  Suwon-si, Gyeonggi-do 16677 (KR)
• NA, Hyoseok
  Suwon-si, Gyeonggi-do 16677 (KR)

(74) Representative: Gulde & Partner
Patent- und Rechtsanwaltskanzlei mbB
Berliner Freiheit 2
10785 Berlin (DE)

(54) ELECTRONIC DEVICE INCLUDING CONDUCTIVE PATTERN FOR PROVIDING SIGNAL

(57) According to embodiments of the present disclosure, a ring-type wearable device is provided. The wearable device includes a housing forming an exterior of the wearable device, a non-conductive structure including a portion exposed to an outside with respect to the housing, wherein the non-conductive structure has a first side on which the portion exposed to the outside is disposed and a second side opposite to the first side, a conductive pattern disposed on the second side of the non-conductive structure, a piezoelectric layer, wherein the piezoelectric layer has a first side coupled with the conductive pattern and a second side opposite to the first side, a connection member disposed on the second side of the piezoelectric layer and connected to the conductive pattern, and a radiator configured to be connected to the connection member. The conductive pattern between the non-conductive structure and the piezoelectric layer is configured to, in a first state prior to receiving a user input that includes contact on the non-conductive structure, reflect a signal component of a first frequency in response to a wireless signal received through the radiator. The conductive pattern between the non-conductive structure and the piezoelectric layer is configured to, in a second state in accordance with the user input on the non-conductive structure, reflect a signal component of a second frequency different from the first frequency, in response to the wireless signal received through the radiator.

FIG. 2

## Description

### [Technical Field]

The following descriptions relate to an electronic device including a conductive pattern for providing signals.

### [Background Art]

A portable communication device may include various types of devices such as a smartphone, a tablet, and a wearable device. Among them, the wearable device is gaining huge popularity as they provide various interactions by connecting to another device such as a smartphone of a user, together with a function to track various biometric parameters such as a heart rate, a sleep pattern, and an activity level. As an alternative wearable formfactor of wearable devices such as a wristwatch, glasses, and clothing, a ring-type wearable device designed to be worn on a finger of the user is being developed.

The above-described information may be provided as a related art for the purpose of helping understanding of the present disclosure. No argument or decision is made as to whether any of the above description may be applied as a prior art related to the present disclosure.

### [Disclosure]

### [Technical Solution]

According to embodiments of the present disclosure, a ring-type wearable device is provided. The wearable device may comprise a housing forming an exterior of the wearable device, a non-conductive structure including a portion exposed to an outside with respect to the housing, wherein the non-conductive structure has a first side on which the portion exposed to the outside is disposed and a second side opposite to the first side, a conductive pattern disposed on the second side of the non-conductive structure, a piezoelectric layer, wherein the piezoelectric layer has a first side coupled with the conductive pattern and a second side opposite to the first side, a connection member disposed on the second side of the piezoelectric layer and connected to the conductive pattern, and a radiator configured to be connected to the connection member. The conductive pattern between the non-conductive structure and the piezoelectric layer may be configured to, in a first state prior to receiving a user input that includes contact on the non-conductive structure, reflect a signal component of a first frequency in response to a wireless signal received through the radiator. The conductive pattern between the non-conductive structure and the piezoelectric layer may be configured to, in a second state in accordance with the user input on the non-conductive structure, reflect a signal component of a second frequency different from the first frequency, in response to the wireless signal received through the radiator.

According to embodiments of the present disclosure, an electronic device is provided. The electronic device may comprise an antenna, a coupler coupled to the antenna, wireless communication circuitry connected to the coupler, at least one processor comprising processing circuitry, and memory storing instructions.

The instructions, when executed by the at least one processor collectively or individually, may cause the electronic device to perform a procedure for paring with a wearable device through the wireless communication circuitry, receive information on a resonant frequency of a conductive pattern of the wearable device from the wearable device through the wireless communication circuitry, transmit a wireless signal corresponding to the resonant frequency through the wireless communication circuitry, determine whether a backscatter signal corresponding to the wireless signal having an intensity lower than a threshold is detected or not, after the backscatter signal having the intensity lower than the threshold is detected, determine whether a user pattern related to a reception of the backscatter signal is detected or not, and in accordance with a determination that the user pattern is detected, execute a function corresponding to the user pattern.

According to embodiments of the present disclosure, a wearable device is provided. The wearable device may comprise a housing defining an exterior of the wearable device, a non-conductive structure coupled to the housing, a piezoelectric layer disposed beneath the non-conductive structure, a conductive portion, interposed between the non-conductive structure and the piezoelectric layer, to contact with the non-conductive structure, and an antenna. The antenna may be connectable to the piezoelectric layer such that a wireless signal received through the antenna from a host device is transmitted to the conductive portion through the piezoelectric layer to cause the conductive portion to transmit a reflection signal while pressure is being applied to the piezoelectric layer through the non-conductive structure.

According to embodiments of the present disclosure, a user input system is provided. The user input system may comprise a host device configured to transmit a wireless signal, and a wearable device. The wearable device may comprise a housing, a non-conductive structure coupled to the housing, a piezoelectric layer disposed beneath the non-conductive structure, a conductive portion, interposed between the non-conductive structure and the piezoelectric layer, to contact with the non-conductive structure, and an antenna connectable to the piezoelectric layer. The wearable device may be configured to receive the wireless signal through the antenna from the host device, and transmit a backscatter signal through the antenna, while the wireless signal is provided to the conductive portion through the piezoelectric layer and pressure is being applied to the piezoelectric layer through the non-conductive structure. The host device may be configured to determine whether a user pattern is detected or not based on whether the backscatter signal having an intensity lower than a threshold is detected or not, and in accordance with a determination that the user pattern is detected, execute a function corresponding to the user pattern.

**[Description of the Drawings]**

FIG. 1 indicates a communication environment of a wearable device.

FIG. 2 indicates an example of a wearable device including a conductive pattern disposed between a non-conductive structure and a piezoelectric layer.

FIG. 3 indicates an example of a wearable device including a conductive pattern disposed between a non-conductive structure and a piezoelectric layer.

FIG. 4 indicates an example of a wearable device including a conductive pattern disposed between a non-conductive structure and a piezoelectric layer.

FIGS. 5A and 5B indicate examples of a conductive pattern.

FIGS. 6A and 6B indicate an example of an electronic device for receiving a user input using a wearable device.

FIG. 7 indicates an example of an electronic device for receiving a user input using a wearable device.

FIG. 8 indicates an operation flow of a wearable device for providing a user input using the wearable device.

FIG. 9 indicates an operation flow of an electronic device for receiving a user input using a wearable device.

FIG. 10 indicates exemplary components of a wearable device.

FIG. 11 is a block diagram of an electronic device in a network environment.

**[Mode for Invention]**

Terms used in the present disclosure are used only to describe a specific embodiment, and may not be intended to limit a range of another embodiment. A singular expression may include a plural expression unless the context clearly means otherwise. Terms used herein, including a technical or a scientific term, may have the same meaning as those generally understood by a person with ordinary skill in the art described in the present disclosure. Among the terms used in the present disclosure, terms defined in a general dictionary may be interpreted as identical or similar meaning to the contextual meaning of the relevant technology and are not interpreted as ideal or excessively formal meaning unless explicitly defined in the present disclosure. In some cases, even terms defined in the present disclosure may not be interpreted to exclude embodiments of the present disclosure.

In various embodiments of the present disclosure described below, a hardware approach will be described as an example. However, since the various embodiments of the present disclosure include technology that uses both hardware and software, the various embodiments of the present disclosure do not exclude a software-based approach.

A term referring to a part an electronic device (e.g., a substrate, a printed circuit board (PCB), a flexible PCB (FPCB), a module, an antenna, an antenna element, circuitry, a processor, a chip, a component, or a device), a term referring to a shape of a part (e.g., a structure, a construction, a support portion, a contact portion, or a protrusion portion), a term referring to a connection portion between structures (e.g., a connection portion, a contact portion, a support portion, a contact structure, a conductive member, or an assembly), a term referring to circuitry (e.g., a PCB, a FPCB, a signal line, a feeding line, a data line, a RF signal line, an antenna line, a RF path, a RF module, RF circuitry, a splitter, a divider, a coupler, or a combiner), and the like, used in the following description are exemplified for convenience of explanation. Therefore, the present disclosure is not limited to terms to be described below, and another term having an equivalent technical meaning may be used. In addition, a term such as '...unit, '...device, '...object', and '...structure', and the like used below may mean at least one shape structure or may mean a unit processing a function.

In addition, in the present disclosure, the term 'greater than' or 'less than' may be used to determine whether a particular condition is satisfied or fulfilled, but this is only a description to express an example and does not exclude description of 'greater than or equal to' or 'less than or equal to'. A condition described as 'greater than or equal to ' may be replaced with 'greater than', a condition described as 'less than or equal to' may be replaced with 'less than', and a condition described as ' greater than or equal to and less than' may be replaced with 'greater than and less than or equal to'. In addition, hereinafter, 'A' to 'B' refers to at least one of elements from A (including A) to B (including B). Hereinafter, 'C' and/or 'D' means including at least one of 'C' or 'D', that is, {'C', 'D', and 'C' and 'D'}.

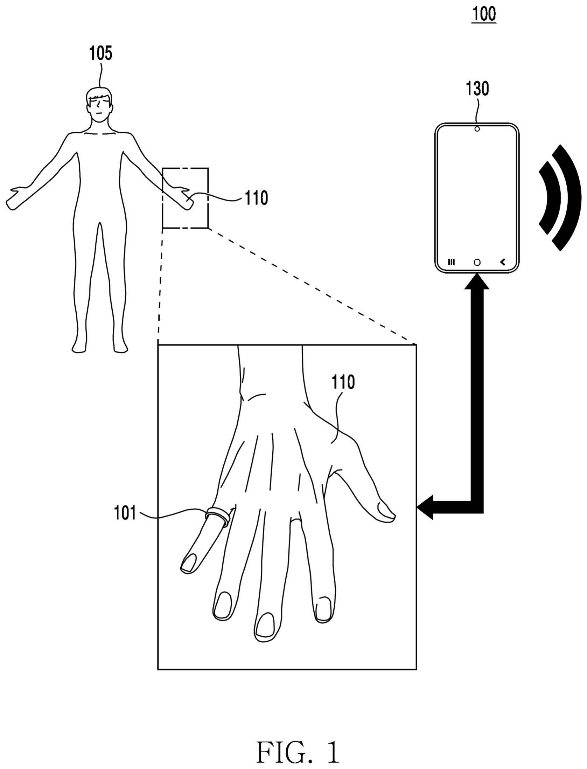

FIG. 1 indicates a communication environment of a wearable device.

Referring to FIG. 1, a communication environment 100 may include a wearable device 101. A user 105 may wear the wearable device 101. For example, the wearable device 101 may be worn on at least a part (e.g., a finger of a hand 110) of a body of the user 105. As an example, the wearable device 101 may be a ring-type device. The wearable device 101 may communicate with an external electronic device 130 (e.g., a smartphone, a smart watch, or another communication device). For example, the wearable device 101 may receive a signal from the external electronic device 130. The wearable device 101 may transmit a signal to the external electronic device 130. The user 105 may control the external electronic device 130 through the wearable device 101. The user 105 may control the external electronic device 130 through a user input on the wearable device 101. A signal transmitted from the wearable device 101 or a pattern (e.g., a change in an intensity) of the signal may be recognized as an input by the external electronic device 130.

As technology develops, a size and power consumption of a device capable of measuring human biometric information (e.g., a body temperature, a blood oxygen concentration, a heart rate, or a breathing pattern) are drastically reduced. The wearable device may receive a user input and transmit the user input to the external electronic device. In order to perform these series of operations, for example, the wearable device may include a touch sensor for obtaining a touch input of a user and circuitry for detecting the touch input that occurs periodically. These components not only require a battery of a larger capacity, but may also be aesthetically disadvantageous for mounting a part in a limited space. Additionally, in a case that the wearable device attempts to transmit a wireless signal indicating a user input to the external electronic device through separate wireless communication circuitry (e.g., bluetooth low energy (BLE) circuitry), power to generate and radiate the wireless signal may be consumed.

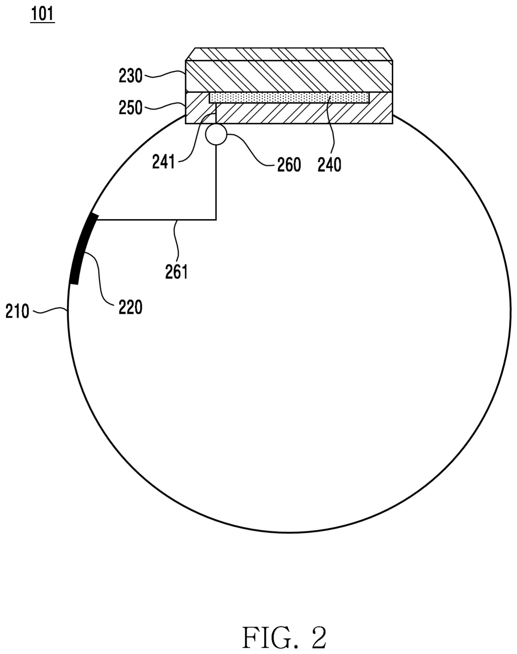

In order to solve the above-described problem, embodiments of the present disclosure may detect a user input through a piezoelectric layer and enable the external electronic device to recognize the user input on the wearable device by reflecting a wireless signal through a conductive pattern on the piezoelectric layer. Hereinafter, examples of the wearable device including the piezoelectric layer and the conductive pattern are described through FIGS. 2 to 4.

FIG. 2 indicates an example of a wearable device (e.g., a wearable device 101) including a conductive pattern disposed between a non-conductive structure and a piezoelectric layer. The same reference number may be used for the same description. The conductive pattern may be referred to as a conductive portion.

Referring to FIG. 2, the wearable device 101 may include a housing 210, a radiator 220 (which can be or act as e.g., an antenna of the wearable device 101, and/or interchangeably referred as an antenna 220 throughout the disclosure), a non-conductive structure 230, a conductive pattern 240, a piezoelectric layer 250, and/or a connection member 260. For example, the wearable device 101 may be a ring-type device. The housing 210, which is a ring-type structure, may have a shape for a finger of a user. The radiator 220 may be a conductive portion used to transmit or receive a wireless signal. For example, the radiator 220 may be a substrate (e.g., a flexible printed circuit board (FPCB) or a plated portion (e.g., laser direct structuring (LDS)) disposed along a circle in the housing 210.

According to an embodiment, the wearable device 101 may include the non-conductive structure 230. The non-conductive structure 230 may include a portion exposed to the outside with respect to the housing 210. As an example, at least a portion of the non-conductive structure 230 may be used as a deco of the wearable device 101. The non-conductive structure 230 may have a first side corresponding to the portion exposed to the outside and a second side opposite to the first side. The second side may not be exposed to the outside. The second side of the non-conductive structure 230 may be coupled with the conductive pattern 240 and the piezoelectric layer 250 to be described later. The non-conductive structure 230 may include a material for transmitting pressure to the piezoelectric layer 250 based on an input (e.g., a pressing input, a touch input, or a rubbing input) of the user. According to an embodiment, the non-conductive structure 230 may include a crystal material (e.g., a material of a crystal type). The non-conductive structure 230 may have a repetitive atomic/molecular structure. For example, the non-conductive structure 230 may be a diamond (e.g., a cubic system). For example, the non-conductive structure 230 may be a ruby or an emerald (e.g., a triclinic system). For example, the non-conductive structure 230 may be a topaz (e.g., a hexagonal system). According to another embodiment, the non-conductive structure 230 may include an amorphous material. For example, the non-conductive structure 230 may include glass. In addition to the above-described examples, the non-conductive structure 230 may include another material. For example, the non-conductive structure 230 may include a material having a hardness level higher than a certain level in order to transmit the pressure in accordance with the input of the user to the piezoelectric layer.

According to various embodiments, the wearable device 101 may be configured to receive a wireless signal from a host device 130 through the antenna, and/or connect the antenna to the piezoelectric layer 250, and/or transmit the wireless signal to the conductive portion 240 through the piezoelectric layer 250, and/or cause the conductive portion 240 to transmit a reflection signal (or transmit a reflection signal using the conductive portion 240). For example, the wearable device 101 may be configured to cause the conductive portion 240 to transmit a reflection signal based on the pressure being applied to the piezoelectric layer 250 through the non-conductive structure 230.

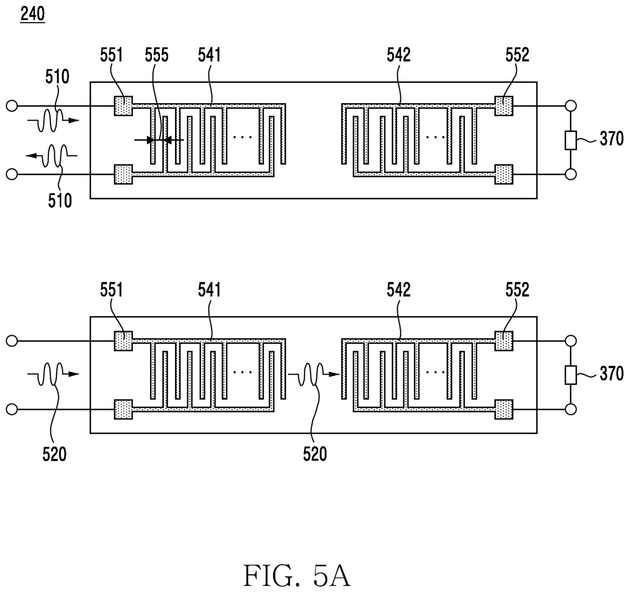

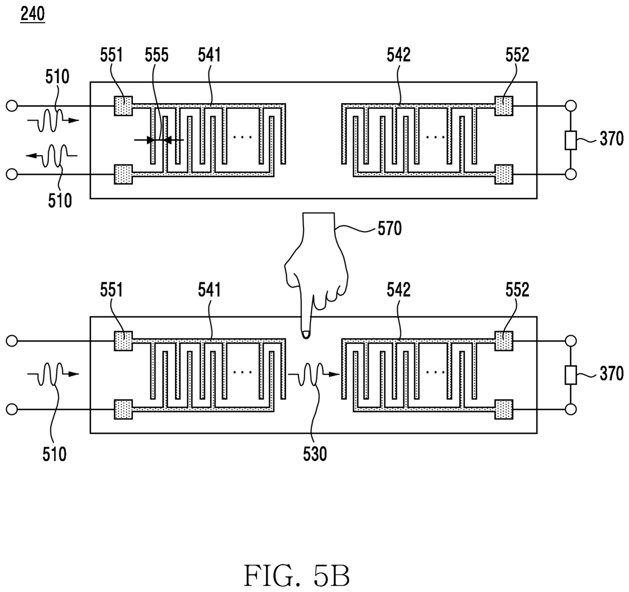

According to an embodiment, the wearable device 101 may include the conductive pattern 240. The conductive pattern 240 may be disposed on the second side of the non-conductive structure 230. As an example, the wearable device 101 may include the piezoelectric layer 250. The piezoelectric layer 250 may be referred to as a piezoelectric plate, a piezoelectric material, a piezoelectric substrate, a piezoelectric element, a piezoelectric film, a piezoelectric structure, piezoelectric circuitry, and/or an equivalent technical/structural term in addition to a piezoelectric layer. The piezoelectric layer 250 may include a first side coupled to the conductive pattern 240 and a second side opposite to the first side. The piezoelectric layer 250 may include a specific material (e.g., zinc oxide or quartz) for converting a wireless signal into a mechanical vibration or converting a mechanical vibration into a wireless signal. The conductive pattern 240 may be disposed between the non-conductive structure 230 and the piezoelectric layer 250. As the non-conductive structure 230 including a specific material (e.g., a crystal material, a crystal type, a diamond, and glass) and/or having a hardness level greater than or equal a certain level, and the piezoelectric layer 250 are bonded to each other, a piezoelectric substrate for transmitting acoustic waves may be formed. The piezoelectric substrate may be understood as a surface acoustic wave (SAW) device. As the conductive pattern 240 is disposed between the non-conductive structure 230 and the piezoelectric layer 250, the conductive pattern 240 may function as an interdigital transducer (IDT) (e.g., aluminum (Al) IDT). The conductive pattern 240 may include a plurality of electrodes. A characteristic of the IDT may be determined in accordance with a gap between electrodes, a width of an electrode, and/or the number of electrodes of the conductive pattern 240. As an example, the electrodes may be arranged to be engaged in a finger shape. For example, the conductive pattern 240 may have a comb shape. A specific shape of the conductive pattern 240 is described in detail through FIGS. 5A and 5B.

According to an embodiment, the wearable device 101 may include the connection member 260. For example, the connection member 260 may be disposed on the second side of the piezoelectric layer 250. The conductive pattern 240 may be electrically connected to the connection member 260 through a first path 241. The connection member 260 may be electrically connected to the radiator 220 through a second path 261. The radiator 220 and the conductive pattern 240 may be electrically connected to each other through the first path 241, the connection member 260, and the second path 261. A wireless signal received through the radiator 220 may be provided to the conductive pattern 240. A wireless signal from the conductive pattern 240 may be transmitted to an external electronic device (e.g., the external electronic device 130) through the radiator 220.

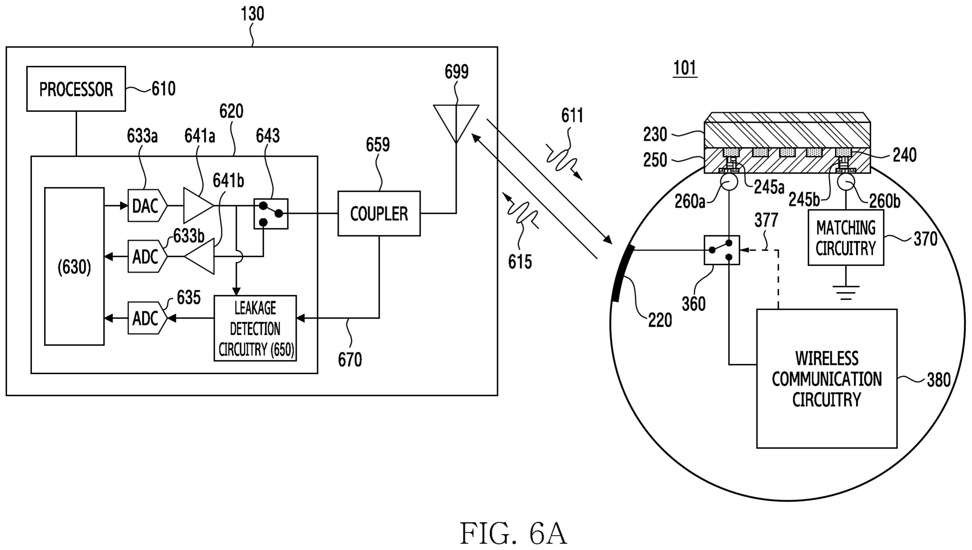

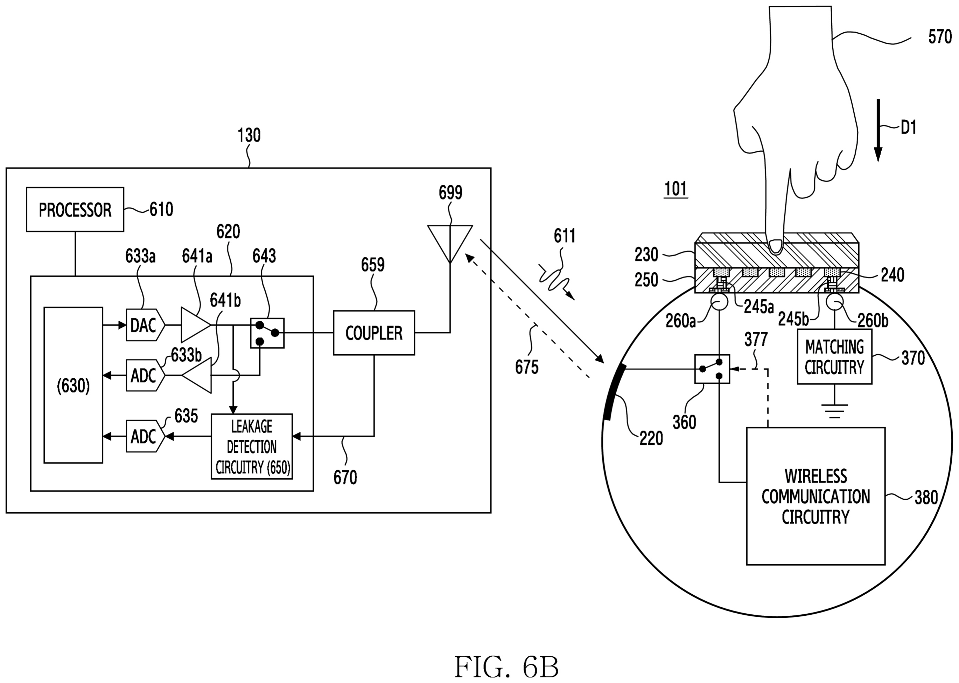

The wearable device 101 according to embodiments of the present disclosure may be used as a control device for providing a user input to the external electronic device 130. A user input on the wearable device 101 may be transmitted to the external electronic device 130. The wearable device 101 may reflect a wireless signal through the conductive pattern 240 on the piezoelectric layer 250 in response to a user input on the non-conductive structure 230. The reflected wireless signal may be transmitted to the external electronic device 130 through the radiator 220. In accordance with a pattern of the reflected wireless signal, the external electronic device 130 may recognize the user input. For example, the conductive pattern 240 may be configured to reflect a wireless signal of the external electronic device 130 with respect to a specific frequency and then no longer reflect the wireless signal of the specific frequency when the user input is received. For example, speed of acoustic waves in the piezoelectric layer 250 may vary in accordance with pressure applied to the piezoelectric layer 250. A resonant frequency in the piezoelectric substrate may vary due to the varying speed. Through the principle, a frequency of the wireless signal of the conductive pattern 240 may vary. The conductive pattern 240 may be configured to reflect a signal component of a first frequency among wireless signals received through the radiator 220. The first frequency may be shifted to a second frequency in accordance with a presence or an absence of a user input through the non-conductive structure 230. In a case that a user input is applied to the non-conductive structure 230, a mechanical vibration characteristic between molecules of the piezoelectric layer 250 may vary. Since the mechanical vibration characteristic between the molecules of the piezoelectric layer 250 varies, a frequency of the signal reflected by the conductive pattern 240 may vary. As the reflected signal varies, the external electronic device 130 may recognize the user input on the wearable device 101. The recognition of the user input in the external electronic device 130 is described in detail through FIGS. 6A and 6B.

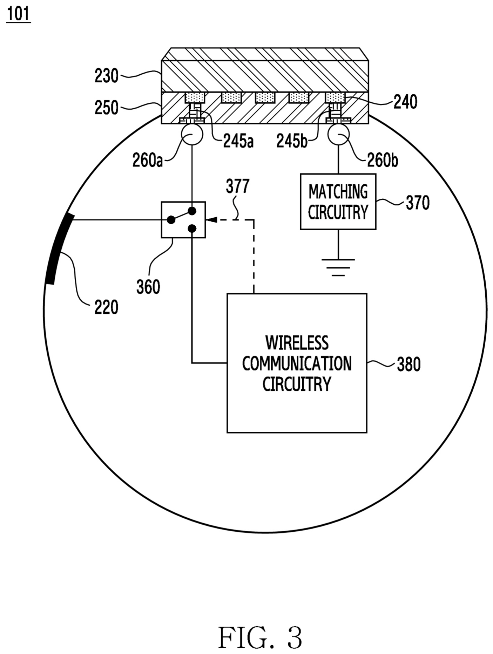

FIG. 3 indicates an example of a wearable device (e.g., a wearable device 101) including a conductive pattern (e.g., a conductive pattern 240) disposed between a non-conductive structure (e.g., a non-conductive structure 230) and a piezoelectric layer (e.g., a piezoelectric layer 250). The same reference number may be used for the same description.

Referring to FIG. 3, the wearable device 101 may include a housing 210, a radiator 220, the non-conductive structure 230, the conductive pattern 240, a first conductive via 245a, a second conductive via 245b, the piezoelectric layer 250, a first connection member 260a, and/or a second connection member 260b. The wearable device 101 may be a ring-type device. For example, the housing 210, which is a ring-type structure, may have a shape for a finger of a user. The descriptions of FIG. 2 may be referenced for the housing 210. The radiator 220 may be a conductive portion used to transmit or receive a wireless signal. The descriptions of FIG. 2 may be referenced for the radiator 220. The non-conductive structure 230 may include a portion exposed to the outside with respect to the housing 210. The descriptions of FIG. 2 may be referenced for the non-conductive structure 230. The conductive pattern 240 may be disposed on a second side of the non-conductive structure 230. The conductive pattern 240 may be disposed between the non-conductive structure 230 and the piezoelectric layer 250. The descriptions of FIG. 2 may be referenced for the conductive pattern 240. The piezoelectric layer 250 may include a first side coupled to the conductive pattern 240 and a second side opposite to the first side. The descriptions of FIG. 2 may be referenced for the piezoelectric layer 250.

According to an embodiment, the first conductive via 245a and/or the second conductive via 245b may be disposed in the piezoelectric layer 250. The first conductive via 245a may connect a first end portion of the conductive pattern 240 to the first connection member 260a. The first connection member 260a may be disposed on the second side of the piezoelectric layer 250. As an example, the first connection member 260a, which is a conductive member for electrically connecting a PCB (not illustrated) to the conductive via 245a, may be a solder bump (or may be referred to as a bump, a solder ball, or soldering). The second conductive via 245b may connect a second end portion of the conductive pattern 240 to the second connection member 260b. The second connection member 260b may be disposed on the second side of the piezoelectric layer 250. As an example, the second connection member 260b, which is a conductive member for electrically connecting the PCB (not illustrated) to the conductive via 245b, may be a solder bump (or may be referred to as a bump, a solder ball, or soldering).

According to an embodiment, the wearable device 101 may include a switching circuit 360. The switching circuit 360 may be configured to selectively connect the radiator 220 to one of the first connection member 260a and wireless communication circuitry 380. For example, in a case that the switching circuit 360 connects the radiator 220 and the first connection member 260a, the conductive pattern 240 may be electrically connected to the radiator 220 through the first conductive via 245a and the first connection member 260a.

According to an embodiment, the wearable device 101 may include matching circuitry 370. The matching circuitry 370 may be connected to the second connection member 260b. The conductive pattern 240 may include resonant patterns. The resonant patterns may include a first resonant pattern and a second resonant pattern. The first resonant pattern may include a plurality of electrodes. The second resonant pattern may include a plurality of electrodes. The first resonant pattern may include the first end portion of the conductive pattern 240. The second resonant pattern may include the second end portion of the conductive pattern 240. The first resonant pattern may be configured to reflect a signal component corresponding to a resonant frequency of the conductive pattern 240 among wireless signals inputted through the first end portion. The first resonant pattern may be configured to transmit a signal component other than the resonant frequency among the wireless signals to the second resonant pattern. For example, the first resonant pattern may convert the signal component into mechanical waves and transmit the mechanical waves to the second resonant pattern. The second resonant pattern may convert the mechanical waves into an electrical signal and transmit the electrical signal to the matching circuitry 370. As the first resonant pattern reflects the signal component of the resonant frequency, the reflected signal component may be radiated to the outside through the switching circuit 360 and the radiator 220. For example, the reflected signal component may be transmitted to an external electronic device (e.g., the external electronic device 130). A user input on the non-conductive structure 230 may change pressure in the piezoelectric layer 250. The change in the pressure may change a mechanical vibration characteristic between molecules. As the pressure in the piezoelectric layer 250 changes, speed of surface acoustic waves propagating through a surface of the piezoelectric layer 250 may vary. The varying speed may cause a change in frequency. If the resonant frequency of the conductive pattern 240 varies, a frequency of a signal component reflected by the conductive pattern 240 may vary. Accordingly, a size and a phase of a signal transmitted to the external electronic device 130 may vary. Since a signal transmitted from the external electronic device 130 mainly includes a signal component of a specific frequency, an intensity of a signal component having a frequency other than the specific frequency may be much smaller than an intensity of a signal component of the specific frequency. Therefore, an intensity of a signal component (e.g., a signal component having a varied frequency) transmitted to the external electronic device 130 after the user input on the non-conductive structure 230 may be smaller than an intensity of a signal component (e.g., the signal component having the specific frequency) transmitted to the external electronic device 130 prior to the user input on the non-conductive structure 230. For example, if the signal component of the specific frequency is repeatedly received and then no longer received, the external electronic device 130 may recognize that a resonant frequency in the wearable device 101 varies. By recognizing that this pattern of signal transmission is changed, the external electronic device 130 may detect a user input from the wearable device 101.

According to various embodiments, the wearable device 101 may be configured to receive a wireless signal from a host device 130 (e.g., the external electronic device 130) through the antenna, and/or connect the antenna to the piezoelectric layer 250, and/or transmit the wireless signal to the conductive portion 240 through the piezoelectric layer 250, and/or cause the conductive portion 240 to transmit a reflection signal (or transmit a reflection signal using the conductive portion 240). For example, the wearable device 101 may be configured to cause the conductive portion 240 to transmit a reflection signal based on the pressure being applied to the piezoelectric layer 250 through the non-conductive structure 230.

According to an embodiment, the wearable device 101 may transmit a wireless signal through the wireless communication circuitry 380. For example, the wearable device 101 may transmit a BLE signal. The wireless communication circuitry 380 may include BLE communication circuitry. For another example, the wearable device 101 may transmit a Wi-Fi signal. The wireless communication circuitry 380 may include wireless LAN communication circuitry. According to an embodiment, the wireless communication circuitry 380 may include control circuitry (e.g., a microcontroller unit (MCU)) for controlling the switching circuit 360. As an example, the wireless communication circuitry 380 may correspond to an integrated chip (IC) including the BLE communication circuitry and the MCU. The MCU of the wireless communication circuitry 380 may transmit a control signal 377 to the switching circuit 360. A connection state of the switching circuit 360 may be controlled in accordance with the control signal 377. The wearable device 101 may selectively perform transmission of a wireless signal using the wireless communication circuitry 380 and transmission of a reflected signal using the conductive pattern 240. The wearable device 101 may control the switching circuit 360 to connect the radiator 220 and the conductive pattern 240 in a first state. The wearable device 101 may control the switching circuit 360 to connect the radiator 220 and the wireless communication circuitry 380 in a second state. As an example, the switching circuit 360 may be a single pole double throw (SPDT).

According to an embodiment, the wearable device 101 may perform BLE communication with the external electronic device 130. The wearable device 101 may perform a BLE connection with the external electronic device 130 through the radiator 220. The switching circuit 360 may connect the radiator 220 and the wireless communication circuitry 380. As a non-limiting example, when the wearable device 101 is booted, the switching circuit 360 may connect the radiator 220 and the wireless communication circuitry 380. While the switching circuit 360 connects the radiator 220 and the wireless communication circuitry 380, the wearable device 101 may continuously monitor a beacon signal. The external electronic device 130 may repeatedly transmit the beacon signal. After searching for the beacon signal, the wearable device 101 may perform a pairing procedure with the external electronic device 130 based on the beacon signal. If there is no data communication for a certain period of time, after a connection in accordance with the pairing procedure is established, the wearable device 101 may operate in a BLE idle mode. In the BLE idle mode, the wearable device 101 may operate in an active state in accordance with a specified cycle to monitor whether a wireless signal is received. In the BLE idle mode, the wearable device 101 may operate in a sleep state to reduce power consumption except for time of operating in the active state. According to an embodiment, the wearable device 101 may electrically connect the radiator 220 and the conductive pattern 240 through the switching circuit 360 in the sleep state. For example, the wearable device 101 may control the switching circuit 360 to connect the radiator 220 and the conductive pattern 240 in the sleep state. If pressure by a user (e.g., a user 105) is applied to the non-conductive structure 230, a resonant frequency with respect to the conductive pattern 240 on the piezoelectric layer 250 may be changed. For example, as a resonant frequency moves from a first frequency to a second frequency, a signal component corresponding to the first frequency may no longer be reflected by the conductive pattern 240. The signal component corresponding to the first frequency may be transmitted to the matching circuitry 370. The wearable device 101 may notify the external electronic device 130 a presence of a user input by changing the resonant frequency in accordance with the user input that provides the pressure to the non-conductive structure 230. The external electronic device 130 may be continuously transmitting a wireless signal, which is the resonant frequency of the conductive pattern 240. The external electronic device 130 may recognize the user input through a change in reception of a backscatter signal corresponding to the wireless signal.

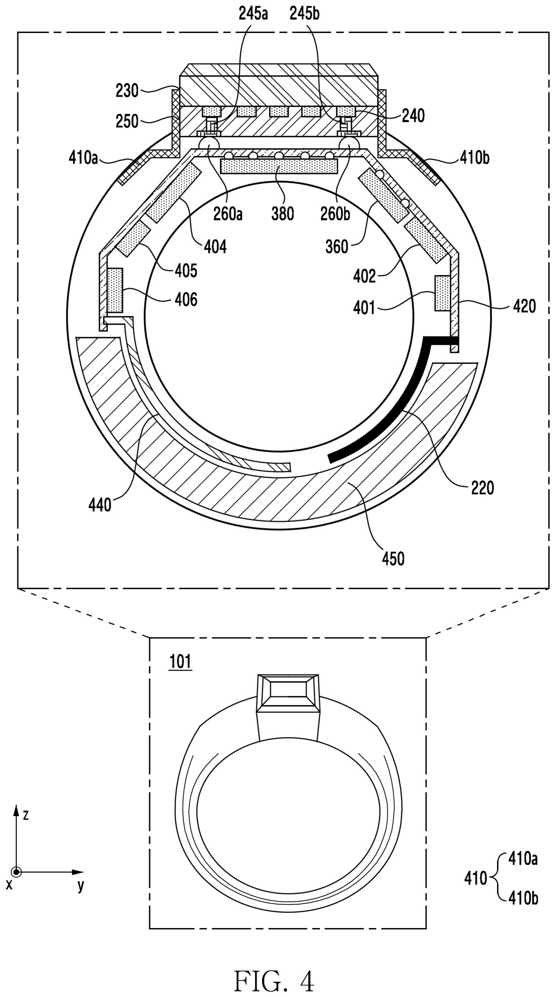

FIG. 4 indicates an example of a wearable device (e.g., a wearable device 101) including a conductive pattern (e.g., a conductive pattern 240) disposed between a non-conductive structure (e.g., a non-conductive structure 230) and a piezoelectric layer (e.g., a piezoelectric layer 250). The same reference number may be used for the same description.

Referring to FIG. 4, the wearable device 101 may include a housing 210, a radiator 220, the non-conductive structure 230, the conductive pattern 240, a first conductive via 245a, a second conductive via 245b, the piezoelectric layer 250, a first connection member 260a, and/or a second connection member 260b. The wearable device 101 may be a ring-type device. For example, the housing 210, which is a ring-type structure, may have a shape for a finger of a user. The descriptions of FIG. 2 may be referenced for the housing 210. The radiator 220 may be a conductive portion used to transmit or receive a wireless signal. The descriptions of FIG. 2 may be referenced for the radiator 220. The non-conductive structure 230 may include a portion exposed to the outside with respect to the housing 210. The descriptions of FIG. 2 may be referenced for the non-conductive structure 230. The conductive pattern 240 may be disposed on a second side of the non-conductive structure 230. The conductive pattern 240 may be disposed between the non-conductive structure 230 and the piezoelectric layer 250. The descriptions of FIG. 2 may be referenced for the conductive pattern 240. The piezoelectric layer 250 may include a first side coupled to the conductive pattern 240 and a second side opposite to the first side. The descriptions of FIG. 2 may be referenced for the piezoelectric layer 250.

According to an embodiment, the wearable device 101 may include a support portion 410. The non-conductive structure 230 may include the portion exposed to the outside. The piezoelectric layer 250 may be connected to a PCB 420 through the first connection member 260a and the second connection member 260b. The support portion 410 may be used to support a laminated structure including the non-conductive structure 230, the conductive pattern 240, and the piezoelectric layer 250. The support portion 410 may be disposed to surround the non-conductive structure 230 and the piezoelectric layer 250. For example, when viewed in a direction (e.g., a (-)x axis direction), the support portion 410 may include a first portion 410a formed on a side and a second portion 410b formed on another side opposite to the side.

According to an embodiment, the wearable device 101 may include the PCB 420. The PCB 420 may include a first side and a second side opposite to the first side. For example, the first connection member 260a and the second connection member 260b may be disposed on the first side of the PCB 420. A plurality of parts may be disposed on the second side of the PCB 420. For example, a three-axis sensor 401, a biometric sensor 402 (e.g., a photoplethysmography (PPG) sensor, or a heart rate monitoring (HRM) sensor), a switching circuit 360, a micro electro mechanical systems (MEMS) sensor 404, a temperature sensor 405, and/or a power management integrated circuit (PMIC) 406 may be disposed on the second side of the PCB 420.

According to an embodiment, the first connection member 260a may be connected to the radiator 220 through the switching circuit 360. For example, the first connection member 260a may be connected to the switching circuit 360 on the second side of the PCB 420 through wiring and a via on the first side of the PCB 420. The switching circuit 360 may be connected to the radiator 220 through the wiring of the PCB 420.

According to an embodiment, the second connection member 260b may be connected to matching circuitry 370. For example, the matching circuitry 370 may be disposed on the first side or the second side of the PCB 420.

As a non-limiting example, the wearable device 101 may include various parts in addition to the above-described components. For example, the wearable device 101 may include a conductive portion 440 for wireless charging. The conductive portion 440 may be referred to as a charging coil, a charging antenna, a conductive coil, and/or an equivalent technical term. As a non-limiting example, the conductive portion 440 may be used as a near-field communication (NFC) coil. For example, the wearable device 101 may include a battery 450 for supplying power.

The wearable device 101 according to embodiments of the present disclosure may be used as a device for controlling the external electronic device 130 in accordance with a user input. Based on a signal reflected through the conductive pattern 240 in the wearable device 101, the wearable device 101 may be used as an input/output device of the user input. Power consumption may be reduced by reflecting a received signal through a physical characteristic of the conductive pattern 240 and the piezoelectric layer 250 without using an additional sensor or wireless communication circuitry. According to an embodiment, the conductive pattern 240 may be electrically connected to the radiator 220 as in FIG. 2 or may be electrically connected to the radiator 220 through the switching circuit 360 as in FIGS. 3 and 4. Therefore, the conductive pattern 240 may be configured to reflect a wireless signal of a resonant frequency of the conductive pattern 240 among wireless signals received through the radiator 220. As an electrical signal corresponding to the wireless signal is supplied to the conductive pattern 240, surface acoustic waves may be generated. The conductive pattern 240 may function as a resonator that totally reflects the electrical signal in accordance with the resonant frequency of the conductive pattern 240, and passes a signal component of a frequency different from the resonant frequency.

FIGS. 5A and 5B indicate examples of a conductive pattern (e.g., the conductive pattern 240). The same reference number may be used for the same description.

Referring to FIG. 5A, a conductive pattern 240 according to an embodiment may include a first resonant pattern 541 and a second resonant pattern 542. The first resonant pattern 541 may include a first end portion 551 for receiving a wireless signal 510. The second resonant pattern 542 may include a second end portion 552 connected to matching circuitry 370.

According to an embodiment, the first resonant pattern 541 may include a plurality of electrodes. The first resonant pattern 541 may be configured to convert an electrical signal into mechanical waves in accordance with a gap 555 between electrodes, a width of each electrode, and/or the number of electrodes.

According to an embodiment, the second resonant pattern 542 may include a plurality of electrodes. The second resonant pattern 542 may be configured to convert mechanical waves into an electrical signal in accordance with a gap between electrodes, a width of each electrode, and/or the number of electrodes. For example, the first resonant pattern 541 may convert the received electrical signal into mechanical waves. The first resonant pattern 541 may transmit the converted mechanical waves to the second resonant pattern 542. The second resonant pattern 542 may convert the mechanical waves into an electrical signal again. In this conversion process, a frequency-selective characteristic may be determined in accordance with an IDT characteristic of the first resonant pattern 541 and/or an IDT characteristic of the second resonant pattern 542.

According to an embodiment, the gap 555 between electrodes may affect a resonant frequency reflected through the first resonant pattern 541. For example, the gap 555 between electrodes may be determined based on the following equation.

<Equation 1>

$$p = \pi v / \omega$$

p indicates a pitch between the electrodes (e.g., a gap between electrode centers points) 555, v indicates propagation speed of surface waves (or surface acoustic waves) on a piezoelectric layer 250, and w indicates a resonant frequency.

The first resonant pattern 541 and/or the second resonant pattern 542 may be configured to reflect a signal component corresponding to a resonant frequency among the wireless signals 510. For example, the first resonant pattern 541 may be configured to reflect the signal component corresponding to the resonant frequency among the wireless signals 510. For example, the second resonant pattern 542 may be configured to reflect the signal component corresponding to the resonant frequency among the wireless signals 510. For example, both the first resonant pattern 541 and the second resonant pattern 542 may be configured to reflect the signal component corresponding to the resonant frequency among the wireless signals 510. A signal component having a frequency different from the resonant frequency among the wireless signals 510 may be transmitted to the matching circuitry 370 through the second resonant pattern 542. For example, if frequencies of the wireless signal 510 are all signals having resonant frequencies, the second resonant pattern 542 may be configured to totally reflect the wireless signal 510. As the wireless signal 510 is totally reflected, the second resonant pattern 542 may not transmit separate mechanical waves to the matching circuitry 370. The second resonant pattern 542 may be configured to reflect a signal component corresponding to a resonant frequency among wireless signals 520. As a non-limiting example, the first resonant pattern 541 may be used for total reflection of the wireless signal 510. As an example, as the wireless signal 510 is totally reflected in the first resonant pattern 542, separate mechanical waves may not be transmitted to the matching circuitry 370. A signal component having a frequency different from the resonant frequency among the wireless signals 520 may be transmitted to the matching circuitry 370. For example, if a frequency of the wireless signal 520 is different from the resonant frequency, the conductive pattern 240 (e.g., the first resonant pattern 541 and/or the second resonant pattern 542) may pass through all of the wireless signals 520.

Referring to FIG. 5B, the conductive pattern 240 may include the first resonant pattern 541 and the second resonant pattern 542. The descriptions of FIG. 5A may be referenced for the first resonant pattern 541 and the second resonant pattern 542. In the piezoelectric layer 250, a molecular bond may be strained due to external pressure. If a mechanical vibration characteristic between molecules are different, speed of acoustic waves may also be different. As the speed changes, a resonant frequency may vary. For example, when referring to Equation 1, the resonant frequency in accordance with the change in the speed may be changed as in the following equation.

<Equation 2>

$$\omega_0 + \Delta\omega = \pi(v + \Delta v)/p$$

$w_0$ indicates a resonant frequency. p indicates the gap 555 between electrodes. v indicates propagation speed of surface waves (or surface acoustic wave) on the piezoelectric layer 250, and $w_0$ indicates the resonant frequency. $\Delta\omega$ indicates an amount of change in the resonant frequency. $\Delta v$ indicates an amount of change in the propagation speed. As deformation is applied to the molecular bond in the piezoelectric layer 250, the propagation speed may vary. Pressure applied to a non-conductive structure 230 due to a user input 570 may be transmitted to the piezoelectric layer 250. The propagation speed is changed due to pressure in the piezoelectric layer 250, and the change in the propagation speed may cause a change in the resonant frequency. For example, assume that the resonant frequency is $w_0$. The wireless signal 510 may be applied to the conductive pattern 240 of a wearable device 101. A frequency of the wireless signal 510 may be $w_0$. In a state before the user input 570 is received, that is, in a free state, a signal component (e.g., the wireless signal 510) corresponding to the w0 frequency may be totally reflected, but in a state in accordance with the user input 570, mechanical waves 530 of the signal component corresponding to the w0 frequency may be transmitted to the matching circuitry 370. This is because the resonant frequency is changed in accordance with the user input 570, and thus w0 is no longer the resonant frequency. The resonant frequency may be $\omega_0 + \Delta\omega$. As the resonant frequency moves, an intensity of a signal of the w0 frequency reflected from the conductive pattern 240 may be reduced.

FIGS. 6A and 6B indicate an example of an electronic device (e.g., an external electronic device 130) for receiving a user input using a wearable device (e.g., a wearable device 101). The same reference number may be used for the same description.

Referring to FIG. 6A, the wearable device 101 may include a housing 210, a radiator 220, a non-conductive structure 230, a conductive pattern 240, a first conductive via 245a, a second conductive via 245b, a piezoelectric layer 250, a first connection member 260a, and/or a second connection member 260b. The wearable device 101 may include a switching circuit 360, matching circuitry 370, and/or wireless communication circuitry 380. The descriptions of FIG. 3 may be referenced for each component of the wearable device 101. The wearable device 101 may perform communication with the external electronic device 130.

According to an embodiment, the external electronic device 130 may include a processor 610, wireless communication circuitry 620, a coupler 659, or an antenna 699 connected to the coupler 659. The wireless communication circuitry 620 may include digital processing circuitry 630 for digital signal processing. The wireless communication circuitry 620 may include a DAC 633a and a power amplifier (PA) 641a for a transmission path. The wireless communication circuitry 620 may include an ADC 633b and a low noise amplifier (LNA) 641b for a reception path. The wireless communication circuitry 620 may include the switching circuit 643 for one of the transmission path and the reception path to the coupler 659. The wireless communication circuitry 620 may include leakage detection circuitry 650 and an ADC 635 for a feedback path 670.

According to an embodiment, the wearable device 101 may operate in a user input mode. The wearable device 101 may control the switching circuit 360 to connect the conductive pattern 240 and the radiator 220 in the user input mode. The external electronic device 130 may transmit a wireless signal 611 having a resonant frequency of the conductive pattern 240 of the wearable device 101. The external electronic device 130 may transmit the wireless signal 611 through the transmission path (e.g., the DAC 633a or the PA 641a), the coupler 659, and the antenna 699. The external electronic device 130 may continuously transmit the wireless signal 611. For example, the external electronic device 130 may periodically transmit the wireless signal 611. If it is in a state before a user input is received by the non-conductive structure 230, while the conductive pattern 240 and the radiator 220 are connected through the switching circuit 360, the conductive pattern 240 of the wearable device 101 may be configured to reflect most of the wireless signal 611. Since a signal incident through the radiator 220 is reflected by the conductive pattern 240, the external electronic device 130 may receive a backscatter signal 615 corresponding to the wireless signal 611.

The external electronic device 130 may receive the backscatter signal 615 through the antenna 699. The backscatter signal 615 received through the antenna 699 may be provided to the leakage detection circuitry 650 through the feedback path 670. In accordance with implementation of circuitry, at least a portion (hereinafter, a leakage signal) of the wireless signal 611 may be leaked and be introduced through the feedback path 670. The leakage detection circuitry 650 may receive a feedback signal. The feedback signal may include the backscatter signal 615 and the leakage signal. The leakage detection circuitry 650 may identify the backscatter signal 615 from the feedback signal and provide the backscatter signal 615 to the ADC 635. For example, the leakage detection circuitry 650 may identify the backscatter signal 615 from the feedback signal based on phase difference information due to a path difference between leakage circuitry and the backscatter signal 615. The leakage detection circuitry 650 may identify the backscatter signal 615 by removing the leakage signal from the feedback signal or reducing a component of the leakage signal. The backscatter signal 615 may be transmitted to the digital processing circuitry 630 and/or the processor 610 through the ADC 635. The external electronic device 130 may recognize a device (e.g., the wearable device 101) configured to reflect the wireless signal 611 by receiving the backscatter signal 615. For example, if the external electronic device 130 periodically transmits the wireless signal 611, the external electronic device 130 may periodically receive the backscatter signal 615. The external electronic device 130 may recognize the wearable device 101 based on the reception of the backscatter signal 615.

Referring to FIG. 6B, the wearable device 101 according to an embodiment may include the conductive pattern 240 disposed between the non-conductive structure 230 and the piezoelectric layer 250. The conductive pattern 240 may be configured to reflect a signal component of a resonant frequency and pass through a signal component of a frequency different from the resonant frequency. A user input 570 may be provided to the non-conductive structure 230 of the wearable device 101. For example, the user input 570 may be an input in which a finger of a user (e.g., a user 105) contacts or presses the non-conductive structure 230. As pressure is applied to the non-conductive structure 230 in a direction D1, the pressure may also be transmitted to the piezoelectric layer 250 coupled to a second side (e.g., a side opposite to a first side exposed to the outside) of the non-conductive structure 230. Propagation speed in the piezoelectric layer 250 may vary in accordance with the pressure in the piezoelectric layer 250. If propagation speed of surface acoustic waves formed on the conductive pattern 240 and the piezoelectric layer 250 varies, a resonant frequency may also vary.

As the resonant frequency varies, a frequency of a reflected signal may also vary. For example, the external electronic device 130 may transmit the wireless signal 611 at a frequency corresponding to a resonant frequency $w_1$ in a state before the user input 570. The frequency of the wireless signal 611 may correspond to $w_1$. Meanwhile, in a state in accordance with the user input 570, the resonant frequency of the conductive pattern 240 may be changed. The resonant frequency of the conductive pattern 240 may be changed from $w_1$ to $w_2$. The conductive pattern 240 may not reflect the wireless signal 611 in the state in accordance with the user input 570. According to an implementation example, the conductive pattern 240 may transmit the backscatter signal 615 at an intensity less than a detection threshold. The wireless signal 611 may be reflected from the conductive pattern 240 with a fine intensity. The conductive pattern 240 may transmit the wireless signal 611 to the matching circuitry 370. As the radiator 220 no longer transmits a backscatter signal to the external electronic device 130 or a backscatter signal 675 with a low intensity is transmitted after the user input 570, it may be difficult for the external electronic device 130 to detect a backscatter signal corresponding to the wireless signal 611.

The external electronic device 130 may monitor a backscatter signal through the leakage detection circuitry 650. The external electronic device 130 may recognize the user input 570 in the wearable device 101 based on a change in a signal characteristic of the backscatter signal. For example, as an amount of the backscatter signal is reduced significantly, the external electronic device 130 may recognize the user input 570 in the wearable device 101. For example, as an amount of change in a phase of the backscatter signal increases significantly, the external electronic device 130 may recognize the user input 570 in the wearable device 101.

In the above-described manner, the wearable device 101 may transmit a presence or an absence of the user input 570 to the external electronic device 130. For example, the user 105 may repeat contact and non-contact with a part (e.g., a finger) of a body of the user 105 to the non-conductive structure 230 in a pattern known to the external electronic device 130. The wearable device 101 may transmit the backscatter signal 615 in accordance with the repetition and then transmit the backscatter signal 675 (a signal transmitted at the intensity less than the detection threshold). The external electronic device 130 may receive the backscatter signal 615 in accordance with the repetition and then receive the backscatter signal 675. In accordance with a reception pattern of a backscatter signal, the external electronic device 130 may recognize a user input corresponding to the reception pattern. The external electronic device 130 may execute a function corresponding to the user input.

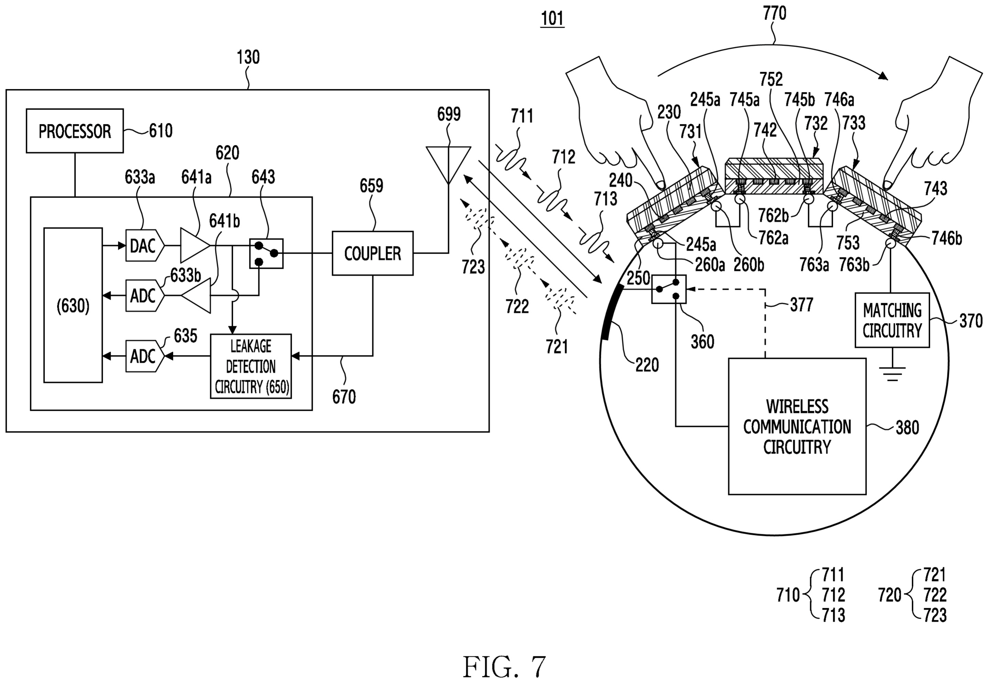

FIG. 7 indicates an example of an electronic device (e.g., an external electronic device 130) for receiving a user input using a wearable device (e.g., a wearable device 101). In FIGS. 6A and 6B, one resonant frequency has been described as an example, but embodiments of the present disclosure are not limited thereto. In FIG. 7, a structure of the wearable device 101 using a plurality of conductive patterns and operations of the external electronic device 130 are described.

Referring to FIG. 7, the wearable device 101 may include a housing 210, a radiator 220, a non-conductive structure 230, a conductive pattern 240, a first conductive via 245a, a second conductive via 245b, a piezoelectric layer 250, a first connection member 260a, and/or a second connection member 260b. The wearable device 101 may include a switching circuit 360, matching circuitry 370, and/or wireless communication circuitry 380. The descriptions of FIG. 3 may be referenced for each component of the wearable device 101. The non-conductive structure 230 may be referred to as a first non-conductive structure. The conductive pattern 240 may be referred to as a first conductive pattern. The piezoelectric layer 250 may be referred to as a first piezoelectric layer. The conductive pattern 240 on the piezoelectric layer 250 may be configured to reflect a signal of a first resonant frequency $w_1$. According to an embodiment, the first connection member 260a may be electrically connected to the radiator 220 through the switching circuit 360. The second connection member 260b may be electrically connected to a third connection member 762a to be described later.

According to an embodiment, the wearable device 101 may include a second non-conductive structure 732, a second conductive pattern (also interchangeably referred to as a second conductive portion throughout the disclosure) 742, and a second piezoelectric layer 752. The second conductive pattern 742 on the second piezoelectric layer 752 may be configured to reflect a signal of a second resonant frequency $w_2$. The second non-conductive structure 732 may include a first side on which a portion exposed to the outside is disposed and a second side opposite to the first side. The second side of the second non-conductive structure 732 may be coupled with a first side of the second piezoelectric layer 752. The second conductive pattern 742 may be disposed on the first side of the second piezoelectric layer 752. The second conductive pattern 742 may be disposed between the second non-conductive structure 732 and the second piezoelectric layer 752. A first end portion of the second conductive pattern 742 may be connected to the third connection member 762a through a third conductive via 745a. A second end portion of the second conductive pattern 742 may be connected to a fourth connection member 762b through a fourth conductive via 745b. The second piezoelectric layer 752 may include the first side and a second side opposite to the first side. The third connection member 762a and the fourth connection member 762b may be disposed on the second side of the second piezoelectric layer 752. According to an embodiment, the third connection member 762a may be electrically connected to the second connection member 260b. The fourth connection member 762b may be electrically connected to a fifth connection member 763a to be described later. As a non-limiting example, each of the third connection member 762a and the fourth connection member 762b may be a solder bump (or may be referred to as a bump, a solder ball, or soldering).

The second non-conductive structure 732 may include a material for transmitting pressure to the second piezoelectric layer 752 based on an input (e.g., a pressing input, a touch input, or a rubbing input) of the user. According to an embodiment, the second non-conductive structure 732 may include a crystal material (e.g., a material of a crystal type). The second non-conductive structure 732 may have a repetitive atomic/molecular structure. For example, the second non-conductive structure 732 may be a diamond (e.g., a cubic system). For example, the second non-conductive structure 732 may be a ruby or an emerald (e.g., a triclinic system). For example, the second non-conductive structure 732 may be a topaz (e.g., a hexagonal system). According to another embodiment, the second non-conductive structure 732 may include an amorphous material. For example, the second non-conductive structure 732 may include glass.

According to an embodiment, the wearable device 101 may include a third non-conductive structure 733, a third conductive pattern 743, and a third piezoelectric layer 753. The third conductive pattern 743 on the third piezoelectric layer 753 may be configured to reflect a signal of a third resonant frequency $w_3$. The third non-conductive structure 733 may include a first side on which a portion exposed to the outside is disposed and a second side opposite to the first side. The second side of the third non-conductive structure 733 may be couple with a first side of the third piezoelectric layer 753. The third conductive pattern 743 may be disposed on the first side of the third piezoelectric layer 753. The third conductive pattern 743 may be disposed between the third non-conductive structure 733 and the third piezoelectric layer 753. A first end portion of the third conductive pattern 743 may be connected to the fifth connection member 763a through a fifth conductive via 746a. A second end portion of the third conductive pattern 743 may be connected to a sixth connection member 763b through a sixth conductive via 746b. The third piezoelectric layer 753 may include the first side and a second side opposite to the first side. The fifth connection member 763a and the sixth connection member 763b may be disposed on the second side of the third piezoelectric layer 753. According to an embodiment, the fifth connection member 763a may be electrically connected to the fourth connection member 762b. The sixth connection member 763b may be electrically connected to the matching circuitry 370. As a non-limiting example, each of the fifth connection member 763a and the sixth connection member 763b may be a solder bump (or may be referred to as a bump, a solder ball, or soldering).

The third non-conductive structure 733 may include a material for transmitting pressure to the third piezoelectric layer 753 based on an input (e.g., a pressing input, a touch input, or a rubbing input) of the user. According to an embodiment, the third non-conductive structure 733 may include a crystal material (e.g., a material of a crystal type). The second the third non-conductive structure 733 may have a repetitive atomic/molecular structure. For example, the third non-conductive structure 733 may be a diamond (e.g., a cubic system). For example, the third non-conductive structure 733 may be a ruby or an emerald (e.g., a triclinic system). For example, the third non-conductive structure 733 may be a topaz (e.g., a hexagonal system). According to another embodiment, the third non-conductive structure 733 may include an amorphous material. For example, the third non-conductive structure 733 may include glass.

According to an embodiment, the wearable device 101 may include a plurality of deco portions (e.g., three deco portions). The deco portions may be electrically connected to each other. For example, the deco portions may be connected in a daisy chain manner. Each deco portion may correspond to a portion of a non-conductive structure exposed to the outside. A user input on each non-conductive structure of the wearable device 101 may change a resonant frequency in a corresponding conductive pattern. For example, if at least a part (e.g., a hand) of a body of a user with respect to the first non-conductive structure 230 is contacted, a resonant frequency in the first conductive pattern 240 on the first piezoelectric layer 250 may be changed. As an example, the resonant frequency in the first conductive pattern 240 may be changed from $w_1$ to $w_4$. For example, if at least a part (e.g., a hand) of a body of a user with respect to the second non-conductive structure 732 is contacted, a resonant frequency in the second conductive pattern 742 on the second piezoelectric layer 752 may be changed. As an example, the resonant frequency in the second conductive pattern 742 may be changed from $w_2$ to $w_5$. For example, if at least a part (e.g., a hand) of a body of a user with respect to the third non-conductive structure 733 is contacted, a resonant frequency in the third conductive pattern 743 on the third piezoelectric layer 753 may be changed. As an example, the resonant frequency in the third conductive pattern 743 may be changed from $w_3$ to $w_6$.

According to an embodiment, the external electronic device 130 may transmit a first wireless signal 711, a second wireless signal 712, and a third wireless signal 713. The external electronic device 130 may transmit the first wireless signal 711, the second wireless signal 712, and the third wireless signal 713 in a time-interleaving manner. For example, the external electronic device 130 may transmit the first wireless signal 711 in a first period, the second wireless signal 712 in a second period after the first period, and the third wireless signal 713 in a third period after the second period. A frequency of the first wireless signal 711 may correspond to $w_1$. A frequency of the second wireless signal 712 may correspond to $w_2$. A frequency of the third wireless signal 713 may correspond to $w_3$.

According to an embodiment, the wearable device 101 may electrically connect the first conductive pattern 240 to the radiator 220 through the switching circuit 360. The first conductive pattern 240 may be configured to reflect a signal component having a frequency corresponding to $w_1$ among wireless signals from the external electronic device 130. The external electronic device 101 may receive a first backscatter signal 721 in accordance with the reflection. A frequency of the first backscatter signal 721 may correspond to $w_1$. The second conductive pattern 742 may be configured to reflect a signal component having a frequency corresponding to $w_2$ among the wireless signals from the external electronic device 130. The external electronic device 101 may receive a second backscatter signal 722 in accordance with the reflection. A frequency of the second backscatter signal 722 may correspond to $w_2$. The third conductive pattern 743 may be configured to reflect a signal component having a frequency corresponding to $w_3$ among the wireless signals from the external electronic device 130. The external electronic device 101 may receive a third backscatter signal 723 in accordance with the reflection. A frequency of the third backscatter signal 723 may

correspond to $w_3$.

According to an embodiment, the external electronic device 130 may recognize an input or an output of the user based on a change in a reception pattern of a reflected signal, for example, backscatter signals. For example, if a finger of the user presses the second non-conductive structure 732, the resonant frequency of the second conductive pattern 742 may be changed from $w_2$ to $w_5$. As the resonant frequency is changed, the second conductive pattern 742 may be configured to reflect a signal component having a frequency different from $w_2$. As a result, the external electronic device 130 may detect that an intensity of a signal having a frequency corresponding to $w_2$ is a threshold. In addition, for example, if the user swipes the portions in an order of the portion exposed from the first non-conductive structure 230 to the outside, the portion exposed from the second non-conductive structure 732 to the outside, and the portion exposed from the third non-conductive structure 733 to the outside, the wearable device 101 may not reflect the first wireless signal 711 having a frequency of $w_1$, then the second wireless signal 712 having a frequency of $w_2$, and then the third wireless signal 713 with a frequency of $w_3$. Accordingly, the external electronic device 130 may recognize a swipe input of the user through a sequential reduction in an intensity of the first backscatter signal 721, an intensity of the second backscatter signal 722 and the third backscatter signal 723 in a received feedback signal.

The three deco portions are illustrated in FIG. 7, but embodiments of the present disclosure are not limited thereto. The wearable device 101 including two deco portions or four or more deco portions may also be understood as an embodiment of the present disclosure. For example, the wearable device 101 may include the first non-conductive structure 230 and the second non-conductive structure 732 corresponding to the two deco portions. Herein, the fourth connection member 762b may be electrically connected to the matching circuitry 370.

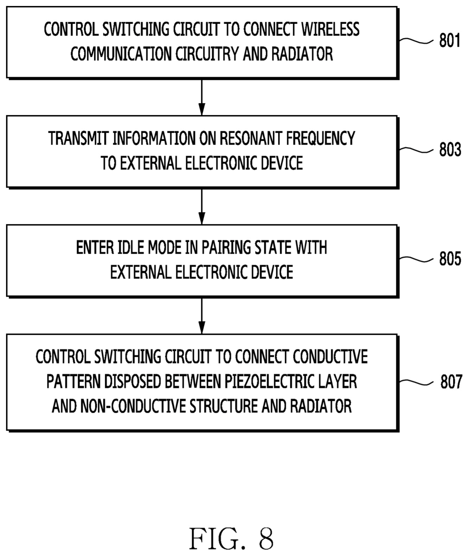

FIG. 8 indicates an operation flow of a wearable device (e.g., a wearable device 101) for providing a user input using the wearable device. The same reference number may be used for the same description.

Referring to FIG. 8, in an operation 801, the wearable device 101 may control a switching circuit (e.g., a switching circuit 360) to connect wireless communication circuitry (e.g., wireless communication circuitry 380) and a radiator (e.g., a radiator 220). As an example, the wearable device 101 may control the switching circuit 360 to connect the wireless communication circuitry 380 and the radiator 220, when being booted. The wireless communication circuitry 380 may be used to transmit or receive a signal for BLE communication. The wearable device 101 may perform a BLE connection procedure with an external electronic device (e.g., an external electronic device 130) through the wireless communication circuitry 380 and the radiator 220.

In an operation 803, the wearable device 101 may transmit information on a resonant frequency to the external electronic device (e.g., the external electronic device 130). For example, the wearable device 101 may transmit the information on the resonant frequency to the external electronic device 130 through the BLE connection. The information on the resonant frequency indicates a resonant frequency in accordance with electrodes of a conductive pattern 240 positioned on a piezoelectric layer (e.g., a piezoelectric layer 250) of the wearable device 101. The resonant frequency indicates a resonant frequency of the conductive pattern 240 in a free state of the wearable device 101, for example, in a state in which there is no user input (e.g., a user input 570) in a deco portion (e.g., a portion of a non-conductive structure 230 exposed to the outside) of the wearable device 101. As an example, the resonant frequency may indicate w.

In an operation 805, the wearable device 101 may enter an idle mode in a pairing state with the external electronic device (e.g., the external electronic device 130). In a case that there is no data to be transmitted or no data to be received for a certain period of time, the wearable device 101 may enter the idle mode (e.g., a BLE idle mode) to reduce battery consumption.

In an operation 807, the wearable device 101 may control the switching circuit (e.g., the switching circuit 360) to connect the conductive pattern (e.g., the conductive pattern 240), disposed between the piezoelectric layer (e.g., the piezoelectric layer 250) and the non-conductive structure (e.g., the non-conductive structure 230), and the radiator (e.g., the radiator 220). The wearable device 101 may be in the BLE idle mode, thereby transmitting a signal in a manner using the conductive pattern 240 and the piezoelectric layer 250. The wearable device 101 may control the switching circuit 360 to connect the radiator 220 and the conductive pattern 240 for the manner. In the operation 803, in accordance with a resonant frequency provided to the external electronic device 130, the external electronic device 130 may transmit a wireless signal at a frequency corresponding to the resonant frequency. The wearable device 101 may receive the wireless signal through the radiator 220. The external electronic device 130 may be configured to reflect a signal corresponding to a current resonant frequency of the conductive pattern 240 among wireless signals received through the radiator 220. For example, in a case of a first state before receiving the user input (e.g., the user input 570), the current resonant frequency of the conductive pattern 240 may be the same as a resonant frequency w transmitted in the operation 803. For example, in a case of a second state in accordance with the user input (e.g., the user input 570), the current resonant frequency of the conductive pattern 240 may be different from the resonant frequency w transmitted in the operation 803. This is because the resonant frequency changes due to a change in speed of surface acoustic waves propagating through a surface of the piezoelectric layer 250 in accordance with pressure of the user input 570. Since the resonant frequency w provided to the external electronic device 130 in the operation 803 is different from the current resonant frequency of the conductive pattern 240, most of the wireless signal may be transmitted to matching circuitry (e.g., matching circuitry 370). As an intensity of a backscatter signal is reduced due to reflection of at least a portion of the wireless signal, the external electronic device 130 may identify that the user input 570 is provided to the wearable device 101.

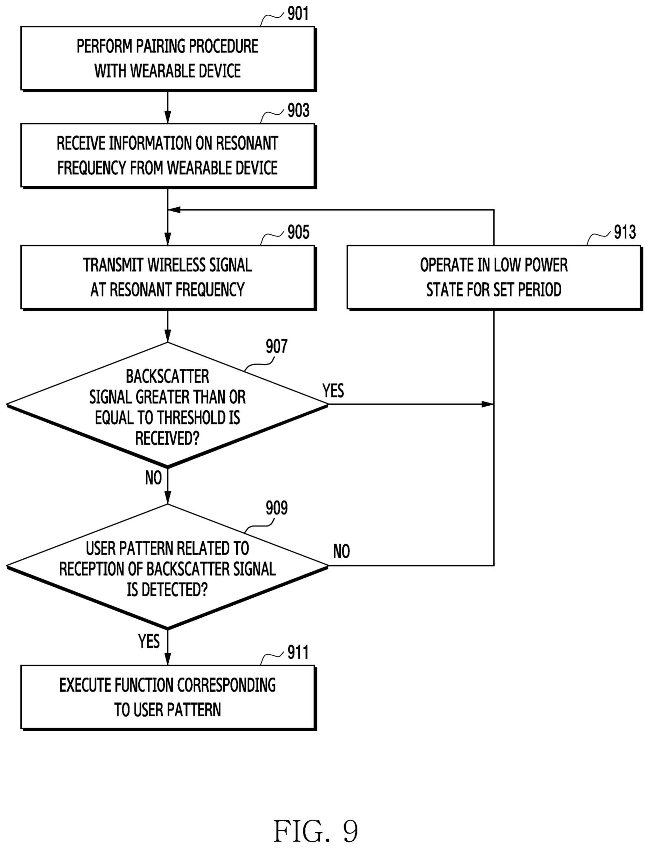

FIG. 9 indicates an operation flow of an electronic device (e.g., an external electronic device 130) for receiving a user input using a wearable device (e.g., a wearable device 101). The same reference number may be used for the same description.

Referring to FIG. 9, in an operation 901, the external electronic device 130 may perform a pairing procedure with the wearable device (e.g., the wearable device 101). For example, the external electronic device 130 may perform the pairing procedure in accordance with BLE communication. The external electronic device 130 may broadcast a beacon signal. By receiving a signal in response to the beacon signal, the external electronic device 130 may perform the pairing procedure with the wearable device 101. As the pairing procedure is completed, a BLE connection between the external electronic device 130 and the wearable device 101 may be established.

In an operation 903, the external electronic device 130 may receive information on a resonant frequency from the wearable device (e.g., the wearable device 101). The external electronic device 130 may receive the information on the resonant frequency from the wearable device 101 through the BLE connection. The information on the resonant frequency indicates a resonant frequency in accordance with electrodes of a conductive pattern 240 positioned on a piezoelectric layer (e.g., a piezoelectric layer 250) of the wearable device 101. The resonant frequency indicates a resonant frequency of the conductive pattern 240 in a free state, for example, when there is no user input (e.g., a user input 570) in a deco portion (e.g., a portion of a non-conductive structure 230 exposed to the outside) of the wearable device 101. The external electronic device 130 may receive the information on the resonant frequency. As an example, the resonant frequency may indicate w.

In an operation 905, the external electronic device 130 may transmit a wireless signal at the resonant frequency w. The external electronic device 130 may enter a BLE idle mode based on identifying that there is no data for a certain period of time or more. The external electronic device 130 may identify w, which is the resonant frequency indicated by information received from the wearable device 101. The external electronic device 130 may transmit a wireless signal (e.g., a wireless signal 611) having the w as a frequency.

In an operation 907, the external electronic device 130 may determine whether a backscatter signal (e.g., a backscatter signal 615 or a backscatter signal 675) greater than or equal to a threshold is received. The wearable device 101 may be configured to reflect a wireless signal of a current resonant frequency of the conductive pattern 240. For example, the wearable device 101 may be configured to reflect a signal component having a frequency corresponding to the resonant frequency w provided in the operation 903. If pressure is applied to the non-conductive structure 230 of the wearable device 101 in accordance with the user input, the resonant frequency of the conductive pattern 240 may vary due to a change in speed in the piezoelectric layer 250. As the resonant frequency of the conductive pattern 240 varies, the conductive pattern 240 may no longer reflect the signal component having the w frequency. As the conductive pattern 240 may reflect a signal of a relatively low intensity or may not reflect any signal by passing through most of the wireless signal having the w frequency. The external electronic device 130 may detect the backscatter signal from a feedback signal in a received feedback path (e.g., a feedback path 670). If an intensity of the backscatter signal is less than the threshold, the external electronic device 101 may recognize that the user input is provided from the wearable device 101. If the intensity of the backscatter signal is greater than or equal to the threshold, the external electronic device 101 may recognize that the user input is prior to being provided (or is not provided) in the wearable device 101. The external electronic device 130 may perform an operation 909 in accordance with a determination that the backscatter signal greater than or equal to the threshold is not received (e.g., the backscatter signal less than the threshold). The external electronic device 130 may perform an operation 913 in accordance with a determination that the backscatter signal greater than or equal to the threshold is received.

In the operation 909, the external electronic device 130 may determine whether a user pattern related to reception of the backscatter signal is detected. As described above in the operation 907, the resonant frequency may vary in accordance with a presence or an absence of the user input on the non-conductive structure 230. A change in a resonant frequency may change an intensity of a reflected signal. Since the external electronic device 130 transmits only a signal of a specific frequency, an intensity of a signal reflected from the conductive pattern 240 may vary in accordance with movement of a resonant frequency. Therefore, the intensity of the backscatter signal received from the external electronic device 130 may also vary.

According to an embodiment, a change pattern of the intensity of the backscatter signal may correspond to the user pattern. As an example, when a user of the wearable device 101 repeats pressing and releasing the non-conductive structure 230 three times, the intensity of the backscatter signal is repeated three times to be smaller and larger than the threshold. Through this pattern related to the reception of the backscatter signal, the external electronic device 101 may detect the user pattern. As a non-limiting example, the user pattern may include a short press input (e.g., less than a certain time (e.g., approximately 2 seconds)) on the non-conductive structure 230, a long press input (e.g., more than the certain time (e.g., approximately 2 seconds)) on the non-conductive structure 230, and/or a double press input on the non-conductive structure 230. The external electronic device 130 may perform an operation 911 in accordance with a determination that the user pattern related to the reception of the backscatter signal is detected. The external electronic device 130 may perform the operation 913 in accordance with a determination that the user pattern related to the reception of the backscatter signal is not detected.

In the operation 911, the external electronic device 130 may execute a function corresponding to the user pattern. The user pattern may indicate that there is the user input in the wearable device 101. For example, the external electronic device 130 may be configured to broadcast the beacon signal in accordance with the user pattern. For example, the external electronic device 130 may attempt a telephone connection to a specific user in accordance with the user pattern. For example, the external electronic device 130 may output a notification signal for notifying a position of the external electronic device 130 in accordance with the user pattern. For example, the external electronic device 130 may execute a camera application and obtain an image in accordance with the user pattern. For example, the external electronic device 130 may perform a series of functions set by the user in accordance with the user pattern.

In the operation 913, the external electronic device 130 may operate in a low power state (e.g., a sleep state) for a set period. Since the external electronic device 130 is in a state of having entered the BLE idle mode after performing the pairing procedure with the wearable device 101, the external electronic device 130 may operate in the sleep state in the BLE idle mode, if there is no separate user input.

In FIG. 9, an example in which the external electronic device 130 identifies the user input on the wearable device 101 in accordance with a change in the intensity of the backscatter signal has been described. However, embodiments of the present disclosure are not limited thereto. The user input on the wearable device 101 may be identified not only through the intensity of the backscatter signal but also through a change in a phase of the backscatter signal. According to an embodiment, instead of the operation 907 of FIG. 9, the wearable device 101 may determine whether a phase change amount of the backscatter signal is greater than or equal to the threshold. The phase change of the backscatter signal may indicate a phase difference between a backscatter signal (hereinafter, a previous backscatter signal) reflected before the user input is applied to the wearable device 101 after a wireless signal is provided from the external electronic device 130 and a currently received backscatter signal. In a case that the phase change amount of the backscatter signal is greater than or equal to the threshold, the external electronic device 130 may determine that the user input is applied to the non-conductive structure 230 of the wearable device 101. The external electronic device 130 may perform the operation 909. In a case that the phase change amount of the backscatter signal is less than the threshold, the external electronic device 130 may determine that there is no user input in the non-conductive structure 230 of the wearable device 101. The external electronic device 130 may perform the operation 913. As a non-limiting example, in determining whether a condition of the operation 907 is satisfied, the external electronic device 130 may consider a condition in accordance with a change in a phase and a condition in accordance with an intensity of a signal together. For example, the external electronic device 130 may determine whether the user input has been applied to the wearable device 101 based on both the phase change amount greater than or equal to the threshold (hereinafter, a phase change amount threshold) and the intensity of the backscatter signal greater than or equal to the threshold. In accordance with a determination that the user input was provided, the external electronic device 130 may perform the operation 909. In accordance with a determination that the user input is not provided, the external electronic device 130 may perform the operation 913.

According to an embodiment, in the operation 909 of FIG. 9, the user pattern of the wearable device 101 may be identified through a change in an intensity of a signal as well the phase change of the backscatter signal. The external electronic device 130 may determine whether the user pattern related to the reception of the backscatter signal is detected. The change in the resonant frequency may change a phase as well as the intensity of the reflected signal. A phase of the signal reflected from the conductive pattern 240 may vary in accordance with the movement of the resonant frequency. Therefore, the phase of the backscatter signal reflected from the external electronic device 130 may vary. A change pattern of the intensity of the backscatter signal may correspond to the user pattern. As an example, when a user of the wearable device 101 repeats pressing and releasing the non-conductive structure 230 three times, the intensity of the backscatter signal is repeated three times to be smaller and larger than the threshold. Through this pattern related to the reception of the backscatter signal, the external electronic device 101 may detect the user pattern. As a non-limiting example, in determining whether a condition of the operation 909 is satisfied, the external electronic device 130 may consider the condition in accordance with a change in a phase and the condition in accordance with an intensity of a signal together. For example, based on both a pattern in accordance with a change in a phase and a pattern in accordance with a change in an intensity of a signal, the external electronic device 130 may determine a pattern of the backscatter signal. The external electronic device 130 may identify the user pattern intended for the user of the wearable device 101 through the determined pattern of the backscatter signal.

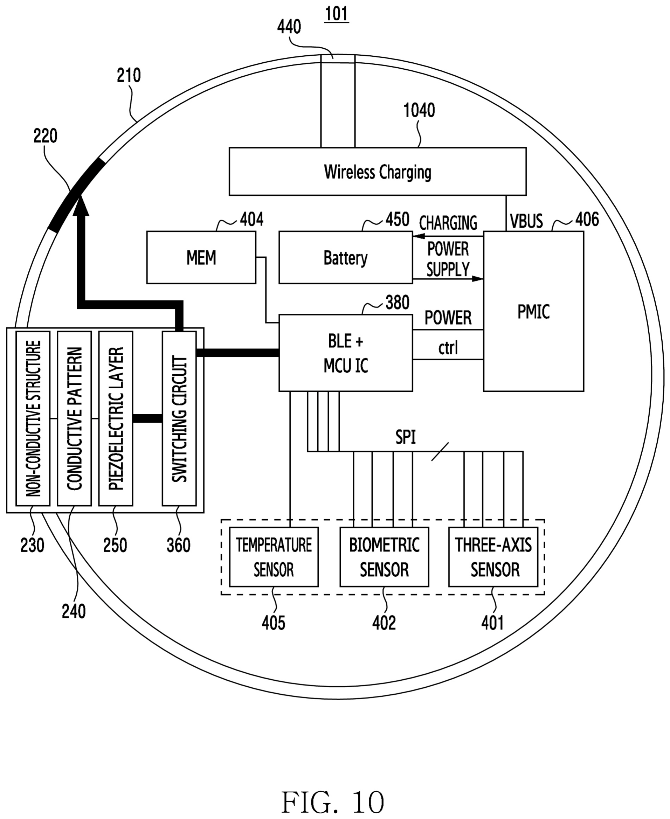

FIG. 10 indicates exemplary components of a wearable device (e.g., a wearable device 101). The same reference number may be used for the same description.

Referring to FIG. 10, the wearable device 101 may include a housing 210, a radiator 220, a non-conductive structure 230, and a conductive pattern 240. A piezoelectric layer 250 may be connected to a switching circuit 360 through a connection member (not illustrated) (e.g., a first connection member 260a or a second connection member 260b). The switching circuit 360 may be connected to the radiator 220. The switching circuit 360 may be configured to selectively electrically connect the radiator to one of the piezoelectric layer 250 and wireless communication circuitry 380 (e.g., BLE +MCU IC).

The wearable device 101 may include various sensors. For example, the wearable device 101 may include a three-axis sensor 401. For example, the wearable device 101 may include a biometric sensor 402 (e.g., a PPG sensor or an HRM sensor). For example, the wearable device 101 may include a micro electro mechanical systems (MEMS) sensor 404. For example, the wearable device 101 may include a temperature sensor 405. As a non-limiting example, the three-axis sensor 401, the biometric sensor 402, and/or the temperature sensor 405 may be connected to the wireless communication circuitry 380 through SPI. A MCU of the wireless communication circuitry 380 may compress data received from at least one of the sensors and store it in memory (not illustrated). The wireless communication circuitry 380 may transmit stored biometric information to an external electronic device 130 through a BLE connection.

The wearable device 101 may include components for power management. For example, the wearable device 101 may include a conductive portion 440 for charging. For example, the wearable device 101 may include a charging IC 1040. For example, the wearable device 101 may include a battery 450. The conductive portion 440 may provide magnetic energy received from external charging circuitry to the charging IC 1040. The charging IC 1040 may process (e.g., rectification and regulation) the magnetic energy and transmit a processed signal to a PMIC 406 as VBUS power. The PMIC 406 may charge the battery 450 based on a current supplied through the VBUS power. The PMIC 406 may be configured to generate power for driving components (e.g., ICs and the wireless communication circuitry 380) in the wearable device 101 and supply the generated power.

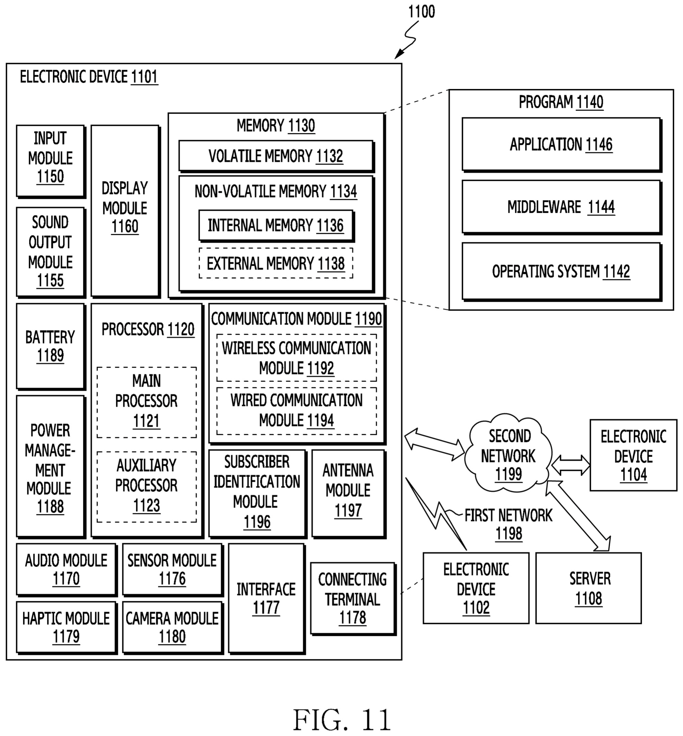

For the components of the external electronic device 130 described through FIGS. 1 to 10, the following descriptions may be referenced. According to an embodiment, the external electronic device 130 may correspond to an electronic device 1101 described in FIG. 11.

As a non-limiting example, at least some of the descriptions of the components of the electronic device 1101 may be referred to as the descriptions of the components of the wearable device 101.

FIG. 11 is a block diagram illustrating an electronic device 1101 in a network environment 1100 according to various embodiments.

Referring to FIG. 11, the electronic device 1101 in the network environment 1100 may communicate with an electronic device 1102 via a first network 1198 (e.g., a short-range wireless communication network), or at least one of an electronic device 1104 or a server 1108 via a second network 1199 (e.g., a long-range wireless communication network). According to an embodiment, the electronic device 1101 may communicate with the electronic device 1104 via the server 1108. According to an embodiment, the electronic device 1101 may include a processor 1120, memory 1130, an input module 1150, a sound output module 1155, a display module 1160, an audio module 1170, a sensor module 1176, an interface 1177, a connecting terminal 1178, a haptic module 1179, a camera module 1180, a power management module 1188, a battery 1189, a communication module 1190, a subscriber identification module(SIM) 1196, or an antenna module 1197. In some embodiments, at least one of the components (e.g., the connecting terminal 1178) may be omitted from the electronic device 1101, or one or more other components may be added in the electronic device 1101. In some embodiments, some of the components (e.g., the sensor module 1176, the camera module 1180, or the antenna module 1197) may be implemented as a single component (e.g., the display module 1160).

The processor 1120 may execute, for example, software (e.g., a program 1140) to control at least one other component (e.g., a hardware or software component) of the electronic device 1101 coupled with the processor 1120, and may perform various data processing or computation. According to an embodiment, as at least part of the data processing or computation, the processor 1120 may store a command or data received from another component (e.g., the sensor module 1176 or the communication module 1190) in volatile memory 1132, process the command or the data stored in the volatile memory 1132, and store resulting data in non-volatile memory 1134. According to an embodiment, the processor 1120 may include a main processor 1121 (e.g., a central processing unit (CPU) or an application processor (AP)), or an auxiliary processor 1123 (e.g., a graphics processing unit (GPU), a neural processing unit (NPU), an image signal processor (ISP), a sensor hub processor, or a communication processor (CP)) that is operable independently from, or in conjunction with, the main processor 1121. For example, when the electronic device 1101 includes the main processor 1121 and the auxiliary processor 1123, the auxiliary processor 1123 may be adapted to consume less power than the main processor 1121, or to be specific to a specified function. The auxiliary processor 1123 may be implemented as separate from, or as part of the main processor 1121.

The auxiliary processor 1123 may control at least some of functions or states related to at least one component (e.g., the display module 1160, the sensor module 1176, or the communication module 1190) among the components of the electronic device 1101, instead of the main processor 1121 while the main processor 1121 is in an inactive (e.g., sleep) state, or together with the main processor 1121 while the main processor 1121 is in an active state (e.g., executing an application). According to an embodiment, the auxiliary processor 1123 (e.g., an image signal processor or a communication processor) may be implemented as part of another component (e.g., the camera module 1180 or the communication module 1190) functionally related to the auxiliary processor 1123. According to an embodiment, the auxiliary processor 1123 (e.g., the neural processing unit) may include a hardware structure specified for artificial intelligence model processing. An artificial intelligence model may be generated by machine learning. Such learning may be performed, e.g., by the electronic device 1101 where the artificial intelligence is performed or via a separate server (e.g., the server 1108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 1130 may store various data used by at least one component (e.g., the processor 1120 or the sensor module 1176) of the electronic device 1101. The various data may include, for example, software (e.g., the program 1140) and input data or output data for a command related thereto. The memory 1130 may include the volatile memory 1132 or the non-volatile memory 1134.

The program 1140 may be stored in the memory 1130 as software, and may include, for example, an operating system (OS) 1142, middleware 1144, or an application 1146.

The input module 1150 may receive a command or data to be used by another component (e.g., the processor 1120) of the electronic device 1101, from the outside (e.g., a user) of the electronic device 1101. The input module 1150 may include, for example, a microphone, a mouse, a keyboard, a key (e.g., a button), or a digital pen (e.g., a stylus pen).

The sound output module 1155 may output sound signals to the outside of the electronic device 1101. The sound output module 1155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 1160 may visually provide information to the outside (e.g., a user) of the electronic device 1101. The display module 1160 may include, for example, a display, a hologram device, or a projector and control circuitry to control a corresponding one of the display, hologram device, and projector. According to an embodiment, the display module 1160 may include a touch sensor adapted to detect a touch, or a pressure sensor adapted to measure the intensity of force incurred by the touch.

The audio module 1170 may convert a sound into an electrical signal and vice versa. According to an embodiment, the audio module 1170 may obtain the sound via the input module 1150, or output the sound via the sound output module 1155 or a headphone of an external electronic device (e.g., an electronic device 1102) directly (e.g., wiredly) or wirelessly coupled with the electronic device 1101.

The sensor module 1176 may detect an operational state (e.g., power or temperature) of the electronic device 1101 or an environmental state (e.g., a state of a user) external to the electronic device 1101, and then generate an electrical signal or data value corresponding to the detected state. According to an embodiment, the sensor module 1176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 1177 may support one or more specified protocols to be used for the electronic device 1101 to be coupled with the external electronic device (e.g., the electronic device 1102) directly (e.g., wiredly) or wirelessly. According to an embodiment, the interface 1177 may include, for example, a high definition multimedia interface (HDMI), a universal serial bus (USB) interface, a secure digital (SD) card interface, or an audio interface.

A connecting terminal 1178 may include a connector via which the electronic device 1101 may be physically connected with the external electronic device (e.g., the electronic device 1102). According to an embodiment, the connecting terminal 1178 may include, for example, an HDMI connector, a USB connector, a SD card connector, or an audio connector (e.g., a headphone connector).

The haptic module 1179 may convert an electrical signal into a mechanical stimulus (e.g., a vibration or a movement) or electrical stimulus which may be recognized by a user via his tactile sensation or kinesthetic sensation. According to an embodiment, the haptic module 1179 may include, for example, a motor, a piezoelectric element, or an electric stimulator.

The camera module 1180 may capture a still image or moving images. According to an embodiment, the camera module 1180 may include one or more lenses, image sensors, image signal processors, or flashes.

The power management module 1188 may manage power supplied to the electronic device 1101. According to an embodiment, the power management module 1188 may be implemented as at least part of, for example, a power management integrated circuit (PMIC).

The battery 1189 may supply power to at least one component of the electronic device 1101. According to an embodiment, the battery 1189 may include, for example, a primary cell which is not rechargeable, a secondary cell which is rechargeable, or a fuel cell.

The communication module 1190 may support establishing a direct (e.g., wired) communication channel or a wireless communication channel between the electronic device 1101 and the external electronic device (e.g., the electronic device 1102, the electronic device 1104, or the server 1108) and performing communication via the established communication channel. The communication module 1190 may include one or more communication processors that are operable independently from the processor 1120 (e.g., the application processor (AP)) and supports a direct (e.g., wired) communication or a wireless communication. According to an embodiment, the communication module 1190 may include a wireless communication module 1192 (e.g., a cellular communication module, a short-range wireless communication module, or a global navigation satellite system (GNSS) communication module) or a wired communication module 1194 (e.g., a local area network (LAN) communication module or a power line communication (PLC) module). A corresponding one of these communication modules may communicate with the external electronic device via the first network 1198 (e.g., a short-range communication network, such as Bluetooth™, wireless-fidelity (Wi-Fi) direct, or infrared data association (IrDA)) or the second network 1199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 1192 may identify and authenticate the electronic device 1101 in a communication network, such as the first network 1198 or the second network 1199, using subscriber information (e.g., international mobile subscriber identity (IMSI)) stored in the subscriber identification module 1196.

The wireless communication module 1192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 1192 may support a high-frequency band (e.g., the mmWave band) to achieve, e.g., a high data transmission rate. The wireless communication module 1192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 1192 may support various requirements specified in the electronic device 1101, an external electronic device (e.g., the electronic device 1104), or a network system (e.g., the second network 1199). According to an embodiment, the wireless communication module 1192 may support a peak data rate (e.g., 20Gbps or more) for implementing eMBB, loss coverage (e.g., 1164dB or less) for implementing mMTC, or U-plane latency (e.g., 0.5ms or less for each of downlink (DL) and uplink (UL), or a round trip of 11ms or less) for implementing URLLC.

The antenna module 1197 may transmit or receive a signal or power to or from the outside (e.g., the external electronic device) of the electronic device 1101. According to an embodiment, the antenna module 1197 may include an antenna including a radiating element composed of a conductive material or a conductive pattern formed in or on a substrate (e.g., a printed circuit board (PCB)). According to an embodiment, the antenna module 1197 may include a plurality of antennas (e.g., array antennas). In such a case, at least one antenna appropriate for a communication scheme used in the communication network, such as the first network 1198 or the second network 1199, may be selected, for example, by the communication module 1190 (e.g., the wireless communication module 1192) from the plurality of antennas. The signal or the power may then be transmitted or received between the communication module 1190 and the external electronic device via the selected at least one antenna. According to an embodiment, another component (e.g., a radio frequency integrated circuit (RFIC)) other than the radiating element may be additionally formed as part of the antenna module 1197.

According to various embodiments, the antenna module 1197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 1101 and the external electronic device 1104 via the server 1108 coupled with the second network 1199. Each of the electronic devices 1102 or 1104 may be a device of a same type as, or a different type, from the electronic device 1101. According to an embodiment, all or some of operations to be executed at the electronic device 1101 may be executed at one or more of the external electronic devices 1102, 1104, or 1108. For example, if the electronic device 1101 should perform a function or a service automatically, or in response to a request from a user or another device, the electronic device 1101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1101. The electronic device 1101 may provide the outcome, with or without further processing of the outcome, as at least part of a reply to the request. To that end, a cloud computing, distributed computing, mobile edge computing (MEC), or client-server computing technology may be used, for example. The electronic device 1101 may provide ultra low-latency services using, e.g., distributed computing or mobile edge computing. In another embodiment, the external electronic device 1104 may include an internet-of-things (IoT) device. The server 1108 may be an intelligent server using machine learning and/or a neural network. According to an embodiment, the external electronic device 1104 or the server 1108 may be included in the second network 1199. The electronic device 1101 may be applied to intelligent services (e.g., smart home, smart city, smart car, or healthcare) based on 5G communication technology or IoT-related technology.

A wearable device 101 according to embodiments of the present disclosure may be used as an input/output (I/O) device for controlling an external electronic device 130. As a manner for recognizing a pattern in accordance with a user input is performed by the external electronic device 130 through reflection of a signal received from the external electronic device 130 other than using power of the wearable device 101, power consumption of the input/output device may be reduced. Since a user 105 wearing the wearable device 101 may touch the wearable device 101 with only movement of a finger in one hand, the user input may be easily provided. In addition, as there is a tendency to add a decoration such as a crystal to the outside, in a case of a ring-type device, the wearable device 101 may have a structure that is easy to provide the user input in accordance with a touch.

The effects that may be obtained from the present disclosure are not limited to those described above, and any other effects not mentioned herein will be clearly understood by those having ordinary knowledge in the art to which the present disclosure belongs, from the following description.

According to embodiments of the present disclosure, a wearable device 101, for example, a ring-type wearable device 101 is provided. The wearable device 101 may comprise a housing 210 forming an exterior of the wearable device 101, a non-conductive structure 230 including a portion exposed to an outside with respect to the housing 210, wherein the non-conductive structure 230 has a first side on which the portion exposed to the outside is disposed and a second side opposite to the first side, a conductive pattern 240 disposed on the second side of the non-conductive structure 230, a piezoelectric layer 250, wherein the piezoelectric layer 250 has a first side coupled with the conductive pattern 240 and a second side opposite to the first side, a connection member disposed on the second side of the piezoelectric layer 250 and connected to the conductive pattern 240, and a radiator 220 configured to be connected to the connection member. The conductive pattern 240 between the non-conductive structure 230 and the piezoelectric layer 250 may be configured to, in a first state prior to receiving a user input that includes contact on the non-conductive structure 230, reflect a signal component of a first frequency in response to a wireless signal received through the radiator 220. The conductive pattern 240 between the non-conductive structure 230 and the piezoelectric layer 250 may be configured to, in a second state in accordance with the user input on the non-conductive structure 230, reflect a signal component of a second frequency different from the first frequency, in response to the wireless signal received through the radiator 220.

For example, the wearable device 101 may comprise a printed circuit board (PCB), wireless communication circuitry 380 disposed on the PCB, and a switching circuit 360 disposed on the PCB. The switching circuit 360 may be configured to connect the radiator 220 to a connection member or the wireless communication circuitry 380 selectively.

For example, the wearable device 101 may comprise matching circuitry disposed on the PCB. The conductive pattern 240 may include a first end portion 551 for receiving RF signals and a second end portion 552 connected to the matching circuitry. The conductive pattern 240 may be configured to reflect a signal component corresponding to a resonant frequency among the RF signals and provide a signal component of a frequency different from the resonant frequency among the RF signal to the matching circuitry. The resonant frequency may correspond to the first frequency in a first state prior to receiving the user input. The resonant frequency may correspond to a second frequency different from the first frequency in a second state in accordance with the user input.

For example, the wearable device 101 may comprise a second connection member disposed on the second side of the piezoelectric layer 250. The connection member may be connected to the first end portion 551 of the conductive pattern 240. The second connection member is connected to the second end portion 552 of the conductive pattern 240. The second connection member may be connected to the matching circuitry.

For example, the wearable device 101 may comprise a first conductive via formed within the piezoelectric layer 250, and a second conductive via formed within the piezoelectric layer 250. The first conductive via may be disposed to connect the first end portion 551 of the conductive pattern 240 and the connection member. The second conductive via may be disposed to connect the second end portion 552 of the conductive pattern 240 and the second connection member.

For example, the conductive pattern 240 may include a first resonant pattern including the first end portion 551, and a second resonant pattern including the second end portion 552. The first resonant pattern and the second resonant pattern may be symmetrically arranged on the non-conductive structure 230.

For example, the switching circuit 360 may be controlled to connect the radiator 220 to the wireless communication circuitry 380 in a Bluetooth low energy (BLE) connection mode with an external electronic device 130. The switching circuit 360 may be controlled to connect the radiator 220 to the connection member in a BLE idle mode.

For example, the wireless communication circuitry 380 may correspond to an integrated chip (IC) including communication circuitry for BLE and a microcontroller unit (MCU).

For example, the piezoelectric layer 250 may comprise a material composed of Zinc Oxide (ZnO).

For example, the conductive portion 240 may include an interdigital transducer (IDT) for converting mechanical waves into an electrical signal or converting an electrical signal into mechanical waves.

For example, a material of the non-conductive portion 230 may correspond to at least one of a crystal material of a crystal type, glass of a crystal type.

For example, the wearable device 101 may comprise a second connection member disposed on the second side of the piezoelectric layer 250, a second non-conductive structure including a portion exposed to an outside with respect to the housing 210, wherein the second non-conductive structure has a first side on which the portion exposed to the outside is disposed and a second side opposite to the first side, a second conductive pattern disposed on the second side of the second non-conductive structure, a second piezoelectric layer, wherein the second piezoelectric layer has a first side coupled to the second conductive pattern and a second side opposite to the first side, a third connection member disposed on the second side of the second piezoelectric layer and connected to the second conductive pattern, a fourth connection member disposed on the second side of the second piezoelectric layer; and matching circuitry connected to the fourth connection member. The second conductive portion may be electrically connected to the first conductive pattern 240 through the second connection member and the third connection member.

For example, the second conductive pattern between the second non-conductive structure and the second piezoelectric layer may be configured to, in a third state prior to receiving a user input that includes contact on the second non-conductive structure, reflect a signal component of a third frequency in response to a wireless signal received through the radiator 220. The second conductive pattern between the second non-conductive structure and the second piezoelectric layer may be configured to, in a fourth state in accordance with the user input on the second non-conductive structure, reflect a signal component of a fourth frequency different from the third frequency in response to the wireless signal received through the radiator 220.

For example, the conductive pattern 240 disposed between the non-conductive structure 230 and the piezoelectric layer 250 may be configured to reflect a signal component of a frequency determined in accordance with pressure in the piezoelectric layer 250 in response to a wireless signal received through the radiator 220.

According to embodiments of the present disclosure, an electronic device 130 is provided. The electronic device 130 may comprise an antenna 699, a coupler 659 coupled to the antenna 699, wireless communication circuitry 620 connected to the coupler 659, at least one processor 610 comprising processing circuitry, and memory storing instructions. The instructions, when executed by the at least one processor 610 collectively or individually, may cause the electronic device 130 to perform a procedure for paring with a wearable device 101 through the wireless communication circuitry 620, receive information on a resonant frequency of a conductive pattern 240 of the wearable device 101 from the wearable device 101 through the wireless communication circuitry 620, transmit a wireless signal corresponding to the resonant frequency through the wireless communication circuitry 620, determine whether a backscatter signal corresponding to the wireless signal having an intensity lower than a threshold is detected or not, after the backscatter signal having the intensity lower than the threshold is detected, determine whether a user pattern related to a reception of the backscatter signal is detected or not, and in accordance with a determination that the user pattern is detected, execute a function corresponding to the user pattern.

For example, the instructions, when executed by the at least one processor 610 collectively or individually, may cause the electronic device 130 to, in a case that the backscatter signal having the intensity lower than the threshold is not detected or the user pattern related to the reception of the backscatter signal is not detected, operate in a low power state for a set period.

For example, the user pattern may indicate a pattern changing from an intensity greater than or equal to the threshold to an intensity less than the threshold.

For example, the instructions, when executed by the at least one processor collectively or individually, may cause the electronic device to, in a case that the backscatter signal having a phase difference with a previous backscatter signal corresponding to the wireless signal after transmitting the wireless signal, that is greater than or equal to a phase change threshold, is not detected or the user pattern related to the reception of the backscatter signal is not detected, operate in a low power state for a set period.

For example, the user pattern may indicate a pattern in which a phase difference of backscatter signals corresponding to the wireless signal changes from being less than the phase change threshold to being greater than or equal to the phase change threshold.

For example, the wireless communication circuitry 620 may include leakage detection circuitry for obtaining the backscatter signal from among a reception signal obtained through the coupler 659. The leakage detection circuitry may be configured to output the backscatter signal from among a transmission leakage signal corresponding to the wireless signal included in the reception signal and the backscatter signal.

For example, the leakage detection circuitry may be configured to apply a cancellation signal utilizing a phase shift of the wireless signal to the reception signal to reduce a component corresponding to the transmission leakage signal in the reception signal. The leakage detection circuitry may be configured to output a result of the application.

For example, the output result may be provided to the at least one processor 610 through an analog to digital converter (ADC) of the wireless communication circuitry 620.

According to embodiments of the present disclosure, a wearable device 101 is provided. The wearable device 101 comprises a housing defining an exterior of the wearable device 101, a non-conductive structure 230 coupled to the housing, a piezoelectric layer 250 disposed beneath the non-conductive structure 230, a conductive portion 240, interposed between the non-conductive structure 230 and the piezoelectric layer 250, to contact with the non-conductive structure 230, and an antenna. The antenna is connectable to the piezoelectric layer 250 such that a wireless signal received through the antenna from a host device 130 is transmitted to the conductive portion 240 through the piezoelectric layer 250 to cause the conductive portion 240 to transmit a reflection signal while pressure is being applied to the piezoelectric layer 250 through the non-conductive structure 230. For example, the wearable device 101 may be configured to receive a wireless signal from a host device 130 through the antenna, and/or connect the antenna to the piezoelectric layer 250, and/or transmit the wireless signal to the conductive portion 240 through the piezoelectric layer 250, and/or cause the conductive portion 240 to transmit a reflection signal (or transmit a reflection signal using the conductive portion 240). For example, the wearable device 101 may be configured to cause the conductive portion 240 to transmit a reflection signal based on the pressure being applied to the piezoelectric layer 250 through the non-conductive structure 230. According to various embodiments of the disclosure, a touch sensor occupying large space in the wearable device may be rendered unnecessary through the arrangement of a relatively more efficient non-conductive structure in conjunction with the particular application of the piezoelectric layer. As such, a lean design for the wearable device may be realized. Moreover, by virtue of the varying characteristics of the reflection signal due to the pressure applied to the piezoelectric layer, the efficiency and accuracy of the user input recognition by the host device can be enhanced. Since a reflection signal is utilized in this manner, it is needless to additionally generate control signal for transmission to the host device, thereby reducing the power consumption of the wearable device.

For example, the signal reflected by the wearable device 101 may be configured to cause the host device 130 to identify that a touch input is applied at the wearable device.

For example, the wearable device 101 may comprise a printed circuit board (PCB), wireless communication circuitry disposed on the PCB, and a switching circuit disposed on the PCB. The switching circuit may be configured to connect the antenna to the conductive portion or the wireless communication circuitry selectively.

For example, the conductive portion 240 may be configured to provide the reflection signal corresponding to a resonant frequency among the RF signals. The resonant frequency may correspond to a first frequency in a case that the wireless signals are received through the antenna while no pressure is applied to the piezoelectric layer 250 through the non-conductive structure 230. The resonant frequency may correspond to a second frequency different from the first frequency in a case that the wireless signals are received through the antenna while pressure is applied to the piezoelectric layer 250 through the non-conductive structure 230.

For example, the switching circuit may be controlled to connect the radiator to the wireless communication circuitry in a Bluetooth low energy (BLE) connection mode with the host device 130. The switching circuit may be controlled to connect the radiator to the connection member while the wireless communication circuitry is in a BLE idle mode. The wireless communication circuitry corresponds to an integrated chip (IC) including communication circuitry for BLE and a microcontroller unit (MCU).

For example, the wearable device 101 may further comprise a first connection member disposed on a second side of the piezoelectric layer 250, that is opposite to a first side of the piezoelectric layer 250 coupled to the non-conductive structure 230, a second connection member disposed on the second side of the piezoelectric layer 250, a first conductive via formed within the piezoelectric layer 250, and a second conductive via formed within the piezoelectric layer 250. The connection member may be connected to a first end portion of the conductive portion 240. The second connection member may be connected to a second end portion of the conductive portion 240. The first conductive via may be disposed to connect the first end portion of the conductive portion 240 and the connection member. The second conductive via may be disposed to connect the second end portion of the conductive portion 240 and the second connection member.

For example, the conductive portion 240 may include a first resonant pattern for obtaining the wireless signals and a second resonant pattern. The first resonant pattern and the second resonant pattern may be symmetrically arranged on the non-conductive structure 230.

For example, the conductive portion 240 between the non-conductive structure 230 and the piezoelectric layer 250 may be configured to, in a first state prior to receiving a user input that includes contact on the non-conductive structure 230, reflect a signal component of a first frequency in response to a wireless signal received through the radiator. The conductive portion 240 between the non-conductive structure 230 and the piezoelectric layer 250 may be configured to, in a second state in accordance with the user input on the non-conductive structure 230, reflect a signal component of a second frequency different from the first frequency, in response to the wireless signal received through the radiator.

For example, the wearable device 101 may comprise a first connection member disposed on a second side of the piezoelectric layer 250, that is opposite to a first side of the piezoelectric layer 250 coupled to the non-conductive structure 230, a second connection member disposed on the second side of the piezoelectric layer 250, a second non-conductive structure 230 coupled to the housing, a second piezoelectric layer 250 disposed beneath the second non-conducive structure 230, a second conductive portion interposed between the second non-conductive structure 230 and the second piezoelectric layer 250, to contact with the second non-conductive structure 230, a third connection member disposed on a second side of the second piezoelectric layer 250, that is opposite to a first side of the second piezoelectric layer 250 coupled to the second non-conductive structure 230, and a fourth connection member disposed on the second side of the second piezoelectric layer 250. The second conductive portion 240 may be electrically connected to the first conductive portion 240 through the second connection member and the third connection member.

For example, the second conductive portion 240 between the second non-conductive structure 230 and the second piezoelectric layer 250 may be configured to, in a third state prior to receiving a user input that includes contact on the second non-conductive structure 230, reflect a signal component of a third frequency in response to a wireless signal received through the radiator. The second conductive portion 240 between the second non-conductive structure 230 and the second piezoelectric layer 250 may be configured to, in a fourth state in accordance with the user input on the second non-conductive structure 230, reflect a signal component of a fourth frequency different from the third frequency in response to the wireless signal received through the radiator.

For example, the conductive portion 240 disposed between the non-conductive structure 230 and the piezoelectric layer 250 may be configured to reflect a signal component of a frequency determined in accordance with pressure in the piezoelectric layer 250 in response to the wireless signal received through the radiator.

According to embodiments of the present disclosure, a user input system is provided. The user input system may comprise a host device configured to transmit a wireless signal, and a wearable device. The wearable device may comprise a housing, a non-conductive structure coupled to the housing, a piezoelectric layer disposed beneath the non-conductive structure, a conductive portion, interposed between the non-conductive structure and the piezoelectric layer, to contact with the non-conductive structure, and an antenna connectable to the piezoelectric layer. The wearable device may be configured to receive the wireless signal through the antenna from the host device, and transmit a backscatter signal through the antenna, while the wireless signal is provided to the conductive portion through the piezoelectric layer and pressure is being applied to the piezoelectric layer through the non-conductive structure. The host device may be configured to determine whether a user pattern is detected or not based on whether the backscatter signal having an intensity lower than a threshold is detected or not, and in accordance with a determination that the user pattern is detected, execute a function corresponding to the user pattern.

For one or more embodiments, at least one of the components described in one or more of the preceding drawings may be configured to perform one or more operations, techniques, processes and/or methods as described in the present disclosure. For example, the processor (e.g., a baseband processor) described in the present disclosure in relation to one or more of the preceding drawings may be configured to operate according to one or more examples described in the present disclosure. For another example, circuitry related to user equipment (UE), a base station, a network element, and the like, as described above in connection with one or more of the previous drawings, may be configured to operate according to one or more examples described herein.

Any of the embodiments described above may be combined with any other embodiment (or combination of embodiments) unless otherwise explicitly stated. The above-described description of one or more implementations provides an example and a description, but is not intended to limit or to exhaust a scope of an embodiment in a precise form disclosed. A modification and a variation are possible in light of the above teachings or may be obtained from practicing various embodiments.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 1140) including one or more instructions that are stored in a storage medium (e.g., internal memory 1136 or external memory 1138) that is readable by a machine (e.g., the electronic device 1101). For example, a processor (e.g., the processor 1120) of the machine (e.g., the electronic device 1101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

**Claims**

1. A wearable device comprising:

    a housing defining an exterior of the wearable device;
    a non-conductive structure coupled to the housing;
    a piezoelectric layer disposed beneath the non-conductive structure;
    a conductive portion, interposed between the non-conductive structure and the piezoelectric layer, to contact with the non-conductive structure; and
    an antenna,
    wherein the antenna is connectable to the piezoelectric layer such that a wireless signal received through the antenna from a host device is transmitted to the conductive portion through the piezoelectric layer to cause the conductive portion to transmit a reflection signal while pressure is being applied to the piezoelectric layer through the non-conductive structure.

2. The wearable device of claim 1,
    wherein the reflection signal is configured to cause the host device to identify that a touch input is applied at the wearable device.

3. The wearable device of any one of claims 1 to 2, further comprising:

    a printed circuit board (PCB);
    wireless communication circuitry disposed on the PCB; and
    a switching circuit disposed on the PCB,
    wherein the switching circuit is configured to connect the antenna to the conductive portion or the wireless communication circuitry selectively.

4. The wearable device of any one of claims 1 to 3,

    wherein the conductive portion is configured to provide the reflection signal corresponding to a resonant frequency among radio frequency (RF) signals, and

wherein the resonant frequency corresponds to:

    a first frequency in a case that wireless signals are received through the antenna while no pressure is applied to the piezoelectric layer through the non-conductive structure, and
    a second frequency different from the first frequency in a case that the wireless signals are received through the antenna while pressure is applied to the piezoelectric layer through the non-conductive structure.

5. The wearable device of any one of claim 3 or 4,

    wherein the switching circuit is controlled to connect the antenna to the wireless communication circuitry in a Bluetooth low energy (BLE) connection mode with the host device,
    wherein the switching circuit is controlled to connect the antenna to the conductive portion while the wireless communication circuitry is in a BLE idle mode, and
    wherein the wireless communication circuitry corresponds to an integrated chip (IC) including communication circuitry for BLE and a microcontroller unit (MCU).

6. The wearable device of any one of claims 1 to 5, further comprising:

    a first connection member disposed on a second side of the piezoelectric layer, that is opposite to a first side of the piezoelectric layer coupled to the non-conductive structure,
    a second connection member disposed on the second side of the piezoelectric layer,
    a first conductive via formed within the piezoelectric layer; and
    a second conductive via formed within the piezoelectric layer,
    wherein the connection member is connected to a first end portion of the conductive portion,
    wherein the second connection member is connected to a second end portion of the conductive portion,
    wherein the first conductive via is disposed to connect the first end portion of the conductive portion and the connection member, and
    wherein the second conductive via is disposed to connect the second end portion of the conductive portion and the second connection member.

7. The wearable device of any one of claims 1 to 6,

    wherein the conductive portion includes a first resonant pattern for obtaining wireless signals and a second resonant pattern, and/or wherein the first resonant pattern and the second resonant pattern are symmetrically arranged on the non-conductive structure, and/or wherein the piezoelectric layer comprises a material composed of Zinc Oxide (ZnO) and/or wherein the conductive portion includes an interdigital transducer (IDT) for converting mechanical waves into an electrical signal or converting an electrical signal into mechanical waves, and/or wherein a material of the non-conductive portion corresponds to at least one of a crystal material of a crystal type, glass of a crystal type.

8. The wearable device of any one of claims 1 to 5, wherein the conductive portion between the non-conductive structure and the piezoelectric layer is configured to:

in a first state prior to receiving a user input that includes contact on the non-conductive structure, reflect a signal component of a first frequency in response to a wireless signal received through the antenna, and in a second state in accordance with the user input on the non-conductive structure, reflect a signal component of a second frequency different from the first frequency, in response to the wireless signal received through the antenna.

9. The wearable device of any one of claims 1 to 5 or 8, further comprising:

a first connection member disposed on a second side of the piezoelectric layer, that is opposite to a first side of the piezoelectric layer coupled to the non-conductive structure; a second connection member disposed on the second side of the piezoelectric layer; a second non-conductive structure coupled to the housing; a second piezoelectric layer disposed beneath the second non-conducive structure; a second conductive portion interposed between the second non-conductive structure and the second piezoelectric layer, to contact with the second non-conductive structure; a third connection member disposed on a second side of the second piezoelectric layer, that is opposite to a first side of the second piezoelectric layer coupled to the second non-conductive structure; a fourth connection member disposed on the second side of the second piezoelectric layer, and wherein the second conductive portion is electrically connected to the first conductive portion through the second connection member and the third connection member.

10. The wearable device of claim 9,

wherein the second conductive portion between the second non-conductive structure and the second piezoelectric layer is configured to:

in a third state prior to receiving a user input that includes contact on the second non-conductive structure, reflect a signal component of a third frequency in response to a wireless signal received through the antenna, and in a fourth state in accordance with the user input on the second non-conductive structure, reflect a signal component of a fourth frequency different from the third frequency in response to the wireless signal received through the antenna, and

wherein the conductive portion disposed between the non-conductive structure and the piezoelectric layer is configured to reflect a signal component of a frequency determined in accordance with pressure in the piezoelectric layer in response to the wireless signal received through the antenna.

11. An electronic device comprising:

an antenna; a coupler coupled to the antenna; wireless communication circuitry connected to the coupler; at least one processor comprising processing circuitry; and memory storing instructions that, when executed by the at least one processor collectively or individually, cause the electronic device to:

perform a procedure for paring with a wearable device through the wireless communication circuitry; receive information on a resonant frequency of a conductive portion of the wearable device from the wearable device through the wireless communication circuitry; transmit a wireless signal corresponding to the resonant frequency through the wireless communication circuitry; determine whether a backscatter signal corresponding to the wireless signal having an intensity lower than a threshold is detected or not, after the backscatter signal having the intensity lower than the threshold is detected, determine whether a user pattern related to a reception of the backscatter signal is detected or not, and,

in accordance with a determination that the user pattern is detected, execute a function corresponding to the user pattern.

12. The electronic device of claim 11, wherein the instructions, when executed by the at least one processor individually or collectively, cause the electronic device to:

in a case that the backscatter signal having the intensity lower than the threshold is not detected or the user pattern related to the reception of the backscatter signal is not detected, operate in a low power state for a set period, and

wherein the user pattern indicates a pattern changing from an intensity greater than or equal to the threshold to an intensity less than the threshold.

13. The electronic device of any one of claim 11 or 12, wherein the instructions, when executed by the at least one processor individually or collectively, cause the electronic device to:

in a case that the backscatter signal having a phase difference with a previous backscatter signal corresponding to the wireless signal after transmitting the wireless signal, that is greater than or equal to a phase change threshold, is not detected or the user pattern related to the reception of the backscatter signal is not detected, operate in a low power state for a set period, and

wherein the user pattern indicates a pattern in which a phase difference of backscatter signals corresponding to the wireless signal changes from being less than the phase change threshold to being greater than or equal to the phase change threshold.

14. The electronic device of any one of claims 11 to 13,

wherein the wireless communication circuitry includes leakage detection circuitry for obtaining the backscatter signal from among a reception signal obtained through the coupler, and

wherein the leakage detection circuitry is configured to output the backscatter signal from among a transmission leakage signal corresponding to the wireless signal included in the reception signal and the backscatter signal, and/or

wherein the leakage detection circuitry is configured to:

apply a cancellation signal utilizing a phase shift of the wireless signal to the reception signal to reduce a component corresponding to the transmission leakage signal in the reception signal, and

output a result of the application.

15. A user input system comprising:

a host device configured to transmit a wireless signal;

a wearable device comprising:

a housing,

a non-conductive structure coupled to the housing,

a piezoelectric layer disposed beneath the non-conductive structure,

a conductive portion, interposed between the non-conductive structure and the piezoelectric layer, to contact with the non-conductive structure; and

an antenna connectable to the piezoelectric layer,

wherein the wearable device is configured to:

receive the wireless signal through the antenna from the host device, and

transmit a backscatter signal through the antenna, while the wireless signal is provided to the conductive portion through the piezoelectric layer and pressure is being applied to the piezoelectric layer through the non-conductive structure, and

wherein the host device is configured to:
determine whether a user pattern is detected or not based on whether the backscatter signal having an intensity lower than a threshold is detected or not, and

in accordance with a determination that the user pattern is detected, execute a function corresponding to the user pattern.

FIG. 1

FIG. 2

FIG. 3

FIG. 4

FIG. 5A

FIG. 5B

FIG. 6A

FIG. 6B

EP 4 730 087 A1

FIG. 7

EP 4 730 087 A1

```
┌─────────────────────────────────────────────┐
│ CONTROL SWITCHING CIRCUIT TO CONNECT WIRELESS │ ⌐ 801
│   COMMUNICATION CIRCUITRY AND RADIATOR        │
└─────────────────────────────────────────────┘
                      │
                      ▼
┌─────────────────────────────────────────────┐
│ TRANSMIT INFORMATION ON RESONANT FREQUENCY    │ ⌐ 803
│      TO EXTERNAL ELECTRONIC DEVICE            │
└─────────────────────────────────────────────┘
                      │
                      ▼
┌─────────────────────────────────────────────┐
│ ENTER IDLE MODE IN PAIRING STATE WITH         │ ⌐ 805
│        EXTERNAL ELECTRONIC DEVICE             │
└─────────────────────────────────────────────┘
                      │
                      ▼
┌─────────────────────────────────────────────┐
│ CONTROL SWITCHING CIRCUIT TO CONNECT CONDUCTIVE│ ⌐ 807
│ PATTERN DISPOSED BETWEEN PIEZOELECTRIC LAYER  │
│ AND NON-CONDUCTIVE STRUCTURE AND RADIATOR     │
└─────────────────────────────────────────────┘
```

FIG. 8

```
               ┌──────────────────────────────┐ ⌐901
               │   PERFORM PAIRING PROCEDURE    │
               │     WITH WEARABLE DEVICE       │
               └──────────────────────────────┘
                              │
                              ▼
               ┌──────────────────────────────┐ ⌐903
               │  RECEIVE INFORMATION ON RESONANT │
               │  FREQUENCY FROM WEARABLE DEVICE │
               └──────────────────────────────┘
                              │
                              ▼
  ┌──────────────────────────┐ ⌐905       ┌──────────────────────────┐ ⌐913
  │   TRANSMIT WIRELESS SIGNAL │          │    OPERATE IN LOW POWER    │
  │     AT RESONANT FREQUENCY  │          │    STATE FOR SET PERIOD    │
  └──────────────────────────┘           └──────────────────────────┘
                │
                ▼
          ╱─────────────╲ ⌐907
         ╱   BACKSCATTER  ╲      YES
        ╱ SIGNAL GREATER THAN OR╲────────►
        ╲ EQUAL TO THRESHOLD IS ╱
         ╲    RECEIVED?    ╱
          ╲─────────────╱
                │ NO
                ▼
          ╱─────────────╲ ⌐909
         ╱ USER PATTERN RELATED TO ╲   NO
        ╱ RECEPTION OF BACKSCATTER SIGNAL╲──────►
        ╲     IS DETECTED?     ╱
          ╲─────────────╱
                │ YES
                ▼
  ┌──────────────────────────┐ ⌐911
  │ EXECUTE FUNCTION CORRESPONDING │
  │      TO USER PATTERN       │
  └──────────────────────────┘
```

FIG. 9

FIG. 10

1100

ELECTRONIC DEVICE 1101

INPUT MODULE 1150

SOUND OUTPUT MODULE 1155

DISPLAY MODULE 1160

MEMORY 1130

VOLATILE MEMORY 1132

NON-VOLATILE MEMORY 1134

INTERNAL MEMORY 1136

EXTERNAL MEMORY 1138

BATTERY 1189

PROCESSOR 1120

MAIN PROCESSOR 1121

AUXILIARY PROCESSOR 1123

POWER MANAGE-MENT MODULE 1188

COMMUNICATION MODULE 1190

WIRELESS COMMUNICATION MODULE 1192

WIRED COMMUNICATION MODULE 1194

SUBSCRIBER IDENTIFICATION MODULE 1196

ANTENNA MODULE 1197

AUDIO MODULE 1170

SENSOR MODULE 1176

HAPTIC MODULE 1179

CAMERA MODULE 1180

INTERFACE 1177

CONNECTING TERMINAL 1178

PROGRAM 1140

APPLICATION 1146

MIDDLEWARE 1144

OPERATING SYSTEM 1142

SECOND NETWORK 1199

ELECTRONIC DEVICE 1104

FIRST NETWORK 1198

ELECTRONIC DEVICE 1102

SERVER 1108

FIG. 11

**TRANSLATION**

## INTERNATIONAL SEARCH REPORT

| International application No. |
|---|
| **PCT/KR2025/012793** |

| A. | CLASSIFICATION OF SUBJECT MATTER |
|---|---|
| | **G06F 3/01**(2006.01)i; **H01Q 1/27**(2006.01)i; **A61B 5/00**(2006.01)i; **A61B 5/024**(2006.01)i; **G06F 1/16**(2006.01)i; **H01Q 5/335**(2015.01)i; **H01Q 5/10**(2015.01)i |

According to International Patent Classification (IPC) or to both national classification and IPC

| B. | FIELDS SEARCHED |
|---|---|

Minimum documentation searched (classification system followed by classification symbols)

G06F 3/01(2006.01); A61B 5/00(2006.01); G01N 29/02(2006.01); G01N 29/036(2006.01); H01L 23/64(2006.01); H01Q 1/38(2006.01); H03H 3/08(2006.01); H03H 9/145(2006.01); H03H 9/64(2006.01); H05K 1/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (MOIP internal) & keywords: 도전성 패턴(conductive pattern), 웨어러블(wearable), 하우징(housing), 압전(piezoelectric), 안테나(antenna), 비도전성(nonconductive), 압력(pressure), 반사(reflection)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | US 2019-0239805 A1 (EPITRONIC HOLDINGS PTE. LTD.) 08 August 2019 (2019-08-08) paragraphs [0083], [0098]; claims 1-2, 20, 24; and figure 13 | 1-3,6-7 |
| A | | 4-5,8-10 |
| Y | KR 10-2014-0001789 A (TAIYO YUDEN CO., LTD.) 07 January 2014 (2014-01-07) paragraphs [0022], [0038]; and figures 1, 6 | 1-3,6-7 |
| A | US 2024-0213958 A1 (RF360 EUROPE GMBH) 27 June 2024 (2024-06-27) paragraphs [0004]-[0017]; and claims 1-23 | 1-10 |
| A | KR 10-2024-0045939 A (SAMSUNG ELECTRONICS CO., LTD.) 08 April 2024 (2024-04-08) paragraphs [0005]-[0006]; and claims 1-15 | 1-10 |
| A | CN 113740422 A (XIAN JIAOTONG UNIVERSITY) 03 December 2021 (2021-12-03) paragraphs [0004]-[0024]; and claims 1-10 | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 January 2026** | **07 January 2026** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2025/012793**

| Box No. III | Observations where unity of invention is lacking (Continuation of item 3 of first sheet) |
|---|---|

This International Searching Authority found multiple inventions in this international application, as follows:

The invention of group 1: claims 1-10 pertain to the structure of a wearable device,
The invention of group 2: claims 11-15 pertain to detecting a user pattern by means of an electronic device, thereby executing a function corresponding to the user pattern.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-10**

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.

**PCT/KR2025/012793**

| Patent document cited in search report | | | Publication date (day/month/year) | Patent family member(s) | | | Publication date (day/month/year) |
|---|---|---|---|---|---|---|---|
| US | 2019-0239805 | A1 | 08 August 2019 | CN | 109690945 | A | 26 April 2019 |
| | | | | EP | 3482495 | A1 | 15 May 2019 |
| | | | | EP | 3482495 | B1 | 15 April 2020 |
| | | | | ES | 2806975 | T3 | 19 February 2021 |
| | | | | WO | 2018-011697 | A1 | 18 January 2018 |
| KR | 10-2014-0001789 | A | 07 January 2014 | CN | 103516326 | A | 15 January 2014 |
| | | | | CN | 103516326 | B | 09 November 2016 |
| | | | | JP | 2014-011570 | A | 20 January 2014 |
| | | | | JP | 5358724 | B1 | 04 December 2013 |
| | | | | TW | 201407955 | A | 16 February 2014 |
| | | | | TW | I578699 | B | 11 April 2017 |
| | | | | US | 2014-0003196 | A1 | 02 January 2014 |
| | | | | US | 9478213 | B2 | 25 October 2016 |
| US | 2024-0213958 | A1 | 27 June 2024 | CN | 120569898 | A | 29 August 2025 |
| | | | | EP | 4639768 | A1 | 29 October 2025 |
| | | | | KR | 10-2025-0125972 | A | 22 August 2025 |
| | | | | TW | 202441889 | A | 16 October 2024 |
| | | | | US | 12316304 | B2 | 27 May 2025 |
| | | | | US | 2025-0317122 | A1 | 09 October 2025 |
| | | | | WO | 2024-136749 | A1 | 27 June 2024 |
| KR | 10-2024-0045939 | A | 08 April 2024 | CN | 120345130 | A | 18 July 2025 |
| | | | | CO | 2025005487 | A2 | 19 May 2025 |
| | | | | EP | 4593195 | A1 | 30 July 2025 |
| | | | | US | 2025-0226572 | A1 | 10 July 2025 |
| | | | | WO | 2024-071929 | A1 | 04 April 2024 |
| CN | 113740422 | A | 03 December 2021 | None | | | |

Form PCT/ISA/210 (patent family annex) (July 2022)